# EUROPÄISCHE PATENTSCHRIFT

(11) **EP 2 879 582 B1**
(45) Veröffentlichungstag und Bekanntmachung des Hinweises auf die Patenterteilung: **25.03.2020**
(21) Anmeldenummer: 13755979.5
(22) Anmeldetag: 01.08.2013
(51) Int. Cl.: A61B 5/16, G10L 25/63, G10L 25/66, A61B 5/11, A61B 5/00, G06N 3/04, G06N 3/08, G16H 50/20

(54) **VORRICHTUNG, VERFAHREN UND APPLIKATION ZUR ERMITTLUNG EINES AKTUELLEN BELASTUNGSNIVEAUS**
DEVICE, METHOD AND APPLICATION FOR ESTABLISHING A CURRENT LOAD LEVEL
DISPOSITIF, PROCÉDÉ ET APPLICATION DE DÉTERMINATION D'UN NIVEAU DE CHARGE ACTUEL

(30) Priorität: 01.08.2012 DE 102012213618; 17.08.2012 DE 102012214697
(43) Veröffentlichungstag der Anmeldung: 10.06.2015
(73) Patentinhaber: Soma Analytics UG (Haftungsbeschränkt), 83052 Bruckmühl (DE)
(72) Erfinder: SCHNEIDER, Peter, 01239 Dresden (DE); HUBER, Johann, 83052 Bruckmühl (DE); LORENZ, Christopher, 80634 München (DE); MARTIN-SERRANO FERNANDEZ, Diego Alberto, London SW16 6EN (GB)
(74) Vertreter: Greaves Brewster LLP
(86) Internationale Anmeldenummer: PCT/EP2013/066241
(87) Internationale Veröffentlichungsnummer: WO 2014/020134

(56) Entgegenhaltungen:
- EP-A2- 2 014 225
- WO-A2-2010/136786
- WO-A2-2012/085687
- US-A- 6 129 681
- US-A1- 2008 208 016
- US-A1- 2010 123 588
- US-A1- 2011 124 977
- US-A1- 2011 283 190
- US-A1- 2012 036 016
- US-A1- 2012 116 186
- US-B1- 7 613 663

## Beschreibung

Die vorliegende Erfindung betrifft eine Vorrichtung zur Ermittlung eines aktuellen Belastungsniveaus eines Benutzers nach dem Oberbegriff des Anspruchs 1, ein Verfahren zur Ermittlung eines aktuellen Belastungsniveaus, sowie eine Applikation für ein mobiles Endgerät.

### Stand der Technik

Nach dem Oberbegriff des Anspruchs 1 ist ein mobiles Endgerät vorgesehen, das über mindestens einen in das mobile Endgerät integrierten Sensor zur Erzeugung von Signaldaten verfügt sowie eine Mehrzahl von verfügbaren Applikationen zur Benutzung durch den Benutzer aufweist. Zusätzlich ist eine Auswertungseinheit vorgesehen. Die Auswertungseinheit ist insbesondere zur Auswertung von Signaldaten vorgesehen.

Bei dem mobilen Endgerät gemäß Oberbegriff handelt es sich beispielsweise um ein Smartphone wie ein iPhone oder ein anderes Smartphone, das mit einem Betriebssystem, wie beispielsweise einem iOS- oder Android-Betriebssystem für ein mobiles Endgerät ausgestattet ist, und über einen integrierten Sensor, beispielsweise einen GPS-Sensor zur Ermittlung der aktuellen Position, verfügt. Auf dem mobilen Endgerät sind eine Mehrzahl von Standardapplikationen installiert, wie beispielsweise eine Telefonie-Applikation zum Aufbau und Durchführen eines Telefonats über eine Mobilfunkverbindung und/oder eine Applikation zum Erstellen, Versenden und Empfangen von SMS und/oder eine Browser-Applikation zum Zugriff auf Webseiten im Internet. Daneben erlaubt das Betriebssystem die Installation und Ausführung weiterer Applikationen, die beispielsweise aus einem Onlineshop für mobile Applikationen im Internet heruntergeladen werden können. Die weitere Applikation kann beispielsweise Daten zum aktuellen Standort des mobilen Endgerätes von dem GPS-Sensor anfordern und verarbeiten und über das Mobilfunknetz, beispielsweise über eine GPRS- oder UMTS-Verbindung, an einen zentralen Server übermitteln. Derlei Trackingdaten können auf dem Server gespeichert und in einer im zentralen Server angeordneten Auswertungseinheit ausgewertet werden, beispielsweise um den Benutzer ortsgebundene Dienste zur Verfügung zu stellen, wie beispielsweise einen Ortungsdienst zum Tracking eines Aufenthaltsortes eines Kindes für dessen Eltern, ein sogenannter Childtracker. Ortsgebundene Dienste werden zur Zeit verbreitet am Markt angeboten und stark nachgefragt, wodurch Smartphones, insbesondere in jüngeren Bevölkerungsgruppen, inzwischen eine weite Verbreitung gefunden haben.

Daneben gibt es anders ausgelegte Vorrichtungen und Verfahren, die mit Hilfe spezieller Geräte sensorbasiert spezifische Vitalfunktionen messen und aus den Messwerten ein aktuelles Belastungsniveau bestimmen. So ermittelt beispielsweise der *Stress Monitor (siehe http:*//*www.healthreviser.com*/*content*/*stress-monitor)* über einen am Ohr des Benutzers getragenen Clip die Herzfrequenz des Benutzers und bestimmt an Hand dieses singulären Indikators ein aktuelles Belastungsniveau.

Weitere bislang bekannte Verfahren bestimmen ein aktuelles Belastungsniveau einer Person beispielsweise auf der Basis von Fragebögen. Ein solches Verfahren stellt beispielsweise der *My Mood Monitor (siehe http:*//*www.whatsmym3.com)* auf Basis eines Onlinefragebogens bereit. Derartige Verfahren auf der Basis von Fragebogendaten können bei wiederholter Durchführung helfen, die zeitliche Veränderung eines Belastungsniveaus aufzuzeigen. Insbesondere können sie für von Depression und/oder Stress Betroffene ein Hilfsmittel bereitstellen, den Erfolg von ergriffenen Maßnahmen zur Verbesserung des Belastungsniveaus zu kontrollieren.

Die genannten Vorrichtungen und Verfahren zur Ermittlung eines aktuellen Belastungsniveaus haben den Nachteil, dass das aktuelle Belastungsniveau auf der Basis von sehr wenigen biometrischen Daten bestimmt wird (beispielsweise subjektiven Fragebogendaten oder objektiven Sensordaten, die eine Vitalfunktion messen), so dass keine Kombination verschiedener Kategorien von biometrischen Daten erfolgt. Deshalb lässt sich das aktuelle Belastungsniveau weniger zuverlässig bestimmen als bei der Verwendung einer Vielzahl von unterschiedlichen Kategorien von biometrischen Daten.

Darüber hinaus greifen die vorgenannten sensorbasierten Vorrichtungen auf genau für diesen Anwendungsfall konstruierte spezielle Geräte zurück, die mit integrierten Sensoren ausgestattet sind, welche eine spezifische Kategorie von biometrischen Daten erfassen, wie beispielsweise die Herzfrequenz oder die Körpertemperatur oder eine andere Vitalfunktion. Diese Geräte müssen vom Benutzer beispielsweise am Körper getragen werden, um sein aktuelles Belastungsniveau zu bestimmen. Dies bedeutet einerseits einen erhöhten Material- und Kostenaufwand, da für die Messung einer Vitalfunktion ein eigenes Gerät mit einem Sensor zur Messung genau dieser Vitalfunktion erforderlich ist. Darüber hinaus stellt das Anbringen und Tragen einer speziellen Vorrichtung mit integriertem Sensor am oder in unmittelbarer Nähe des Körpers eine Beeinträchtigung für den Träger, insbesondere eine Einschränkung von Komfort und Wohlbefinden, dar.

Dagegen erfordern die oben genannten fragebogenbasierten Verfahren einen hohen Zusatzaufwand für den Benutzer. So muss der Benutzer zum Beispiel regelmäßig Fragebogendaten beantworten, beispielsweise mit Hilfe eines Computers über das Internet, und dann die zeitlich veränderlichen Ergebnisse selbstständig verwalten, vergleichen und bewerten.

Dokument US2012036016 offenbart eine Vorrichtung zur Ermittlung eines aktuellen Belastungsniveaus eines Benutzers mittels eines Verfahrens basierend auf einem Netz von neuronalen Netzen, die durch eine Mehrzahl von Prozessoren parallel berechnet werden.

### Aufgabenstellung

Aufgabe der vorliegenden Erfindung ist es daher eine Vorrichtung sowie ein Verfahren und eine Applikation der eingangs genannten Art zu schaffen, welche die zuvor genannten Nachteile überwinden. Insbesondere ist es Aufgabe der Erfindung eine Vorrichtung und ein Verfahren, sowie eine Applikation zu schaffen, welche ein aktuelles Belastungsniveau eines Benutzers mit hoher Zuverlässigkeit bestimmen, wobei der Zusatzaufwand des Benutzers hinsichtlich Kosten und Zeit minimiert wird ohne den Komfort des Benutzers einzuschränken, da die Erfindung auf den Einsatz von zusätzlichen, am oder nahe am Körper zu tragenden Geräten und/oder Sensoren verzichtet.

Diese Aufgabe wird erfindungsgemäß durch die Merkmale der Patentansprüche 1, 10 und 14 gelöst. Vorteilhafte Ausgestaltungen der Erfindung finden sich in den Unteransprüchen und in der nachfolgenden Beschreibung.

Die vorliegende Erfindung umfasst eine auf einem mobilen Endgerät installierbare weitere Applikation, die eine Vielzahl von biometrischen Daten eines Benutzers ermittelt. Zur Ermittlung der biometrischen Daten wirkt die weitere Applikation mit anderen Komponenten zusammen, die ebenfalls in dem mobilen Endgerät angeordnet sind. Die anderen Komponenten sind etwa in das mobile Endgerät integrierte Sensoren sowie verfügbare Applikationen. Eine verfügbare Applikation bezeichne eine auf einem mobilen Endgerät verfügbare Anwendung. Das heißt eine Anwendung, die auf dem mobilen Endgerät installiert und ausführbar ist,, wie etwa Telefonie-, SMS-, MMS-, Chat-Applikationen und/oder Browser-Applikationen zum Zugriff auf das Internet sowie andere Applikationen, die zur Extraktion taktiler, akustischer und/oder visueller biometrischer Merkmale des Benutzers geeignet sind.

Bei dem mobilen Endgerät handelt es sich beispielsweise um ein Smartphone oder einen Tablet-Computer oder einen PDA oder ein anderes mobiles Endgerät, welches der Benutzer für vielfältige Zwecke verwenden kann, beispielsweise zur Kommunikation.

Das mobile Endgerät verfügt über Mittel zur Installation und Ausführung einer weiteren Applikation. Beispielsweise kann die weitere Applikation über einen in das Betriebssystem des mobilen Endgerätes integrierten Onlineshop, wie den App Store, oder einen anderen Onlineshop, der für das mobile Endgerät kompatible Applikationen anbietet, über das Internet bezogen und direkt auf dem mobilen Endgerät installiert werden. Die weitere Applikation kann in verschiedenen Ausführungen vorliegen, beispielsweise in einer iOS-Version zur Installation und Ausführung auf einem iPhone oder einem iPad und in einer Android-Version zur Installation und Ausführung auf einem mobilen Endgerät, das dieses Android-Betriebssystem unterstützt, oder in einer weiteren Version, die zu einem weiteren mobilen Betriebssystem kompatibel ist. Die weitere Applikation kann aber auch auf einer austauschbaren Komponente des mobilen Endgerätes installiert sein und ausgeführt werden. Sie kann etwa als SIM-Applikation im Speicherbereich auf einer SIM-Karte, die im mobilen Endgerät betrieben werden kann, gespeichert sein und von einer separaten auf der SIM-Karte integrierten Ausführungseinheit ausgeführt werden.

Durch Installation und Ausführung einer erfindungsgemäßen weiteren Applikation, in einer zum mobilen Endgerät kompatiblen Version, auf einem mobilen Endgerät der eingangs beschriebenen Art, wird der auf dem mobilen Endgerät angeordnete Teil der Vorrichtung zur Ermittlung eines aktuellen Belastungsniveaus erlangt.

Die weitere Applikation ermittelt die biometrischen Daten einerseits aus Signaldaten, die von in das mobile Endgerät integrierten Sensoren erzeugt werden, und andererseits werden biometrische Daten aus den Nutzungsdaten anderer auf dem mobilen Endgerät installierter Applikationen extrahiert.

Die Bestimmung einer Mehrzahl von biometrischen Daten wird zum einen dadurch erleichtert, dass mobile Endgeräte, wie etwa Smartphones, standardmäßig mit einer zunehmenden Zahl von Sensoren ausgestattet werden. Darüber hinaus wird die Bestimmung weiterer biometrischer Daten auch dadurch erleichtert, dass Benutzer ihre Interaktions- und Kommunikationsbedürfnisse zunehmend durch Nutzung solcher Geräte befriedigen und sich somit etwa biometrische Daten zu den Kategorien Sprache und soziale Interaktion direkt, und ohne Mehraufwand und Komforteinschränkung für den Benutzer, aus Nutzungsdaten zu Kommunikations-Applikationen des mobilen Endgerätes ableiten lassen.

Beispielsweise können aus den Nutzungsdaten einer auf dem mobilen Endgerät installierten Telefonie-Applikation, insbesondere aus den Sprachdaten des Benutzers, die über das Mikrofon des mobilen Handgerätes ermittelt werden und die von dem mobilen Endgerät beispielsweise über ein Funknetzwerk an den Kommunikationspartner übertragen werden, von der weiteren Applikation durch eine Sprachanalyse biometrische Daten zur Sprache, wie Lautstärke, Sprachgeschwindigkeit und/oder Modulationsfähigkeit, ermittelt werden. Die Sprachdaten des Benutzers können aber auch aus der Verwendung in anderen verfügbaren Applikationen stammen, beispielsweise aus Applikationen, die über eine Sprachsteuerung vom Benutzer gesteuert werden.

Aus den Nutzungsdaten einer auf dem mobilen Endgerät installierten SMS-Applikation können von der weiteren Applikation die Anzahl der versendeten und empfangenen SMS-Nachrichten und die Anzahl der verschiedenen Empfänger und Versender von SMS-Nachrichten bestimmt werden, und damit biometrische Daten zur sozialen Interaktion bestimmt werden.

Ein in das mobile Endgerät integrierter Sensor kann beispielsweise ein Gyroskop bzw. Kreiselinstrument sein. Ein Gyroskop dient der Positionsbestimmung im Raum und findet in aktuell am Markt angebotenen Smartphones verstärkt Verbreitung, wie beispielsweise im iPhone 4. Mit Hilfe des Gyroskops und weiterer Sensoren, die in das mobile Endgerät integriert sind, wie etwa einem Beschleunigungssensor und/oder einem Lichtsensor, können biometrische Daten zur Schlafstruktur des Benutzers ermittelt werden. Dazu wird das mobile Endgerät nachts auf der Matratze positioniert und die Bewegungen des Benutzers in der Nacht werden durch die Sensoren detektiert. Die weitere Applikation sammelt die von Gyroskop, Beschleunigungs- und/oder Lichtsensor erzeugten Signaldaten und ermittelt daraus biometrische Daten zu Schlafqualität und Schlafverlauf, wie Einschlafzeitpunkt, Schlafdauer und/oder Schlafstadien.

Daneben können auch vom Benutzer beantwortete Fragebogendaten, die beispielsweise über ein Web-Formular in einer Browser-Applikation oder in einem Formular, das die weitere Applikation bereitstellt, abgefragt werden, zu den von der weiteren Applikation ermittelten biometrischen Daten gehören. Da die Beantwortung von Fragebogendaten einen zusätzlichen Aufwand für den Benutzer generiert, sollten diese das subjektive Belastungsniveau wiedergebenden Daten nur einmalig bzw. möglichst selten erfragt werden.

In einer möglichen Ausführungsform werden die biometrischen Daten ständig durch die weitere Applikation ermittelt, während das mobile Endgerät in Betrieb ist. Das mobile Endgerät ist derart konfiguriert, dass die weitere Applikation beim Einschalten des mobilen Endgerätes beispielsweise automatisch gestartet wird und bis zum Ausschalten des mobilen Endgerätes laufend im Hintergrund betrieben wird, ohne dass eine weitere Interaktion mit dem Benutzer notwendig ist. Ebenso könnte das mobile Endgerät derart konfiguriert sein, dass der Benutzer die weitere Applikation selbsttätig aktivieren, konfigurieren und deaktivieren kann, und auf diese Weise regelt, zu welchen Zeiten welche biometrische Benutzerdaten durch die weitere Applikation verfolgt und zur Auswertung bereitgestellt werden sollen.

Ist die weitere Applikation aktiviert, dann werden die von den in das mobile Endgerät integrierten Sensoren erzeugten Signaldaten, die durch die weitere Applikation verfolgt werden, ständig von der weiteren Applikation empfangen und daraus die biometrischen Daten ermittelt. Ebenso werden Nutzungsdaten aus von dem Benutzer benutzten Standardapplikationen, die von der weiteren Applikation verfolgt werden, wie etwa Telefonie- und/oder SMS-Applikationen, ständig von der weiteren Applikation ausgewertet und daraus biometrische Daten bestimmt.

Die durch die weitere Applikation bestimmten biometrischen Daten lassen sich in verschiedene Kategorien unterteilen. Die durch die weitere Applikation ermittelten biometrischen Daten gehören den Kategorien Schlaf, Sprache, Motorik, Soziale Interaktion, Ökonomische Daten, Persönliche Daten sowie Fragebogen-Daten an.

Die von der weiteren Applikation ermittelten biometrischen Daten können durch eine erste Auswertungsvorrichtung, die in der weiteren Applikation vorgesehen ist, auf dem mobilen Endgerät ausgewertet werden. Die von der weiteren Applikation ermittelten biometrischen Daten können aber auch durch eine zweite Auswertungsvorrichtung auf einem zentralen Server ausgewertet werden. Durch die zweite Auswertungsvorrichtung kann die Qualität der Auswertung weiter erhöht werden.

Die von der weiteren Applikation ermittelten biometrischen Daten werden mit Standardmitteln, die das mobile Endgerät bereitstellt, vom mobilen Endgerät an einen zentralen Server übertragen. Die Übertragung der biometrischen Daten erfolgt pseudonymisiert. Dazu wird der Benutzer auf dem zentralen Server unter einem Pseudonym, und nicht unter seinem echten Namen oder einem anderen, den Benutzer identifizierenden Identifikator, geführt. Die übertragenen biometrischen Daten werden dem Benutzer über das Pseudonym zugeordnet. Um die Sicherheit weiter zu erhöhen, werden die biometrischen Daten verschlüsselt übertragen.

Für die Übertragung werden die zu übertragenden Daten von der weiteren Applikation an eine auf dem mobilen Endgerät angeordnete Übertragungseinheit übergeben. Die Übertragungseinheit ist für Aufbau, Erhaltung und Abbau einer Verbindung zu einem Netzwerk zuständig, beispielsweise einer GPRS- oder UMTS-Verbindung zu einem Mobilfunknetzwerk, an das die Daten übertragen werden. Eine Übertragung erfolgt nur, wenn sich das mobile Endgerät im Übertragungsmodus befindet. Die Übertragungseinheit könnte auch für eine Übertragung über verschiedene Netzwerke ausgelegt sein, beispielsweise ein oder mehrere Mobilfunknetzwerk(e) und/oder eine drahtlose Verbindung zu einem lokalen Netzwerk, beispielsweise nach einem IEEE-802 Standard, wie eine WLAN- oder eine WPAN-Verbindung. Über ein Übertragungsnetzwerk, beispielsweise ein Mobilfunknetzwerk, und ggf. weitere mit diesem Übertragungsnetzwerk über beispielsweise Gateways verbundene Netzwerke, wie etwa dem Internet, über die der zentrale Server erreichbar ist, werden die Daten an den zentralen Server übertragen.

Daneben besteht die Möglichkeit, die von der weiteren Applikation ermittelten Daten zunächst auf einer lokalen, auf dem mobilen Endgerät angeordneten Speichereinheit zu speichern, bevor die Daten an den zentralen Server übertragen werden. Dies ist insbesondere dann notwendig, wenn die Übertragungseinheit zeitweise nicht in der Lage ist, die Daten über ein Übertragungsnetzwerk zu übermitteln. Beispielsweise wenn keine Verbindung zu einem Übertragungsnetzwerk besteht, etwa wenn sich das mobile Endgerät im Flugmodus befindet und damit die Übertragungseinheit deaktiviert ist oder wenn die Übertragungseinheit keine Verbindung zu einem Übertragungsnetzwerk aufbauen kann, da sich das mobile Endgerät außerhalb des Übertragungsbereiches eines Übertragungsnetzwerks befindet.

Die biometrischen Daten können auch direkt von der weiteren Applikation auf dem mobilen Endgerät analysiert und ausgewertet werden. In dieser Ausführungsform ermittelt die weitere Applikation selbstständig aus den biometrischen Daten ein aktuelles Belastungsniveau des Benutzers.

Der zentrale Server verfügt über eine Empfangseinheit, über die Daten aus dem Netzwerk, beispielsweise aus dem Internet, empfangen werden können. Werden die biometrischen Daten des Benutzers vom mobilen Endgerät an den zentralen Server übertragen, so empfängt die Empfangseinheit diese Daten und speichert sie in einer zentralen Speichereinheit.

Die gespeicherten Daten werden an eine auf dem zentralen Server angeordnete Auswertungseinheit übergeben und durch die Auswertungseinheit analysiert und ausgewertet. Die übertragenen und gespeicherten Daten umfassen eine Mehrzahl biometrischer Daten zu einem Benutzer, die von der Auswertungseinheit benutzt werden, um ein aktuelles Belastungsniveau für den Benutzer zu ermitteln.

Da das aktuelle Belastungsniveau eine individuelle Größe darstellt und beispielsweise keinen Vergleich mit objektiven Normwerten beinhaltet, stellt das in dieser Anmeldung offenbarte Verfahren kein Diagnoseverfahren dar. Vielmehr handelt es sich um ein Verfahren, das biometrische Daten zu einem Benutzer ermittelt, sammelt und analysiert sowie die Ergebnisse der Analyse dem Benutzer in Form mindestens eines aktuellen Belastungsniveaus zur Verfügung stellt.

Die Datenanalyse dient dabei insbesondere dazu eine Veränderung des mindestens einen Belastungsniveaus festzustellen; d.h. festzustellen, ob sich das mindestens eine aktuelle Belastungsniveau im Vergleich zu einem vorherigen Belastungsniveau erhöht oder verringert hat. Dem Benutzer wird damit ein Hilfsmittel geboten, insbesondere über zeitliche Änderungen seines mindestens einen aktuellen Belastungsniveaus im Vergleich zu früheren Belastungsniveaus informiert zu werden und nach selbstständiger Bewertung der festgestellten Änderungen ggf. individuelle Maßnahmen zur Stressreduktion zu ergreifen.

Zur Ermittlung eines aktuellen Belastungsniveaus eines Benutzers kann die Auswertungseinheit beispielsweise auf in der Vergangenheit ermittelte biometrische Daten zum Benutzer zurückgreifen und bei der Ermittlung dieses aktuellen Belastungsniveaus berücksichtigen. So können in der Vergangenheit ermittelte biometrische Daten zum Benutzer als Referenzdaten verwendet werden um eine nutzerspezifische Kalibrierung vorzunehmen. Die Ermittlung des aktuellen Belastungsniveaus erfolgt in Bezug auf die vorliegenden Referenzdaten.

Ebenso kann die Auswertungseinheit bei der Ermittlung eines aktuellen Belastungsniveaus neben den biometrischen Daten des Benutzers auf biometrische Daten anderer Nutzer zurückgreifen. Auf diese Weise können beispielsweise Cluster von Benutzergruppen gebildet werden, beispielsweise nach Alter, Geschlecht oder Beruf. Zur Ermittlung des aktuellen Belastungsniveaus eines Benutzers können die Daten von anderen Benutzern derselben Benutzergruppe berücksichtigt werden.

Erfindungsgemäß, ermittelt die Auswertungseinheit ein aktuelles Belastungsniveau eines Benutzers mit Hilfe von künstlichen neuronalen Netzen. Die künstlichen neuronalen Netze werden auf Basis der vorliegenden biometrischen Daten einer Vielzahl von Benutzern trainiert. Durch das Training lernt das künstliche neuronale Netz sukzessive dazu und kann dadurch die Qualität des ermittelten aktuellen Belastungsniveaus weiter verbessern.

Das künstliche neuronale Netz kann beispielsweise auf der Basis eines Mehrschicht-Perceptron-Netzes realisiert werden. Dieses neuronale Netz besteht aus mehreren Schichten, einer festen Eingabeschicht und einer festen Ausgabeschicht und ggf. weiteren dazwischen liegenden Schichten, wobei keine Rückkopplung von einer Schicht auf davor liegende Schichten stattfindet.

Das künstliche neuronale Netz kann beispielsweise aus genau drei Schichten Eingabeschicht, Verborgener Schicht und Ausgabeschicht bestehen. Dabei können beispielsweise die sieben Kategorien von biometrischen Daten Schlaf, Sprache, Motorik, Soziale Interaktion, Ökonomische Daten, Persönliche Daten sowie Fragebogen-Daten die Neuronen der Eingabeschicht bilden. In einer anderen Ausführungsform wird mit einer größeren Anzahl von Neuronen in der Eingabeschicht gearbeitet, indem feiner granulare Kategorien-Merkmale als Neuronen der Eingabeschicht verwendet werden. In einer anderen Ausführungsform verfügt das künstliche neuronale Netz über Rückkopplungsmechanismen. Verfügt das künstliche neuronale Netz über Rückkopplungsmechanismen, so wird es mehrfach (iterativ) durchlaufen.

Erfindungsgemäß, ermittelt die Auswertungseinheit ein aktuelles Belastungsniveau eines Benutzers mit Hilfe eines Netzes von künstlichen neuronalen Netzen, beispielsweise mithilfe eines Deep Belief Networks. Das Netz von künstlichen neuronalen Netzen setzt sich aus einer Mehrzahl von neuronalen Netzen zusammen, die miteinander interagieren. In einem Netz von neuronalen Netzen umfasst ein einzelnes neuronales Netz eine Eingabeschicht und eine verborgene Schicht. Es ist eine erste Ebene von neuronalen Netzen vorgesehen. In den neuronalen Netzen der ersten Ebene ist die Eingabeschicht durch die biometrischen Daten des Benutzers festgelegt. Beispielsweise ist die Eingabeschicht eines neuronalen Netzes der ersten Ebene durch die biometrischen Daten des Benutzers genau einer Kategorie festlegbar. Aus der Eingabeschicht eines künstlichen neuronalen Netzes ist die verborgene Schicht desselben neuronalen Netzes bestimmbar. Darüber hinaus ist eine zweite und ggf. weitere Ebene von neuronalen Netzen vorgesehen. Für ein neuronales Netz der zweiten und jeder weiteren Ebene ist die Eingabeschicht über die verborgenen Schichten einer Mehrzahl von neuronalen Netzen der vorausgehenden Ebene bestimmbar. Das aktuelle Belastungsniveau lässt sich aus mindestens einem neuronalen Netz einer obersten Ebene bestimmen.

Durch die Vielzahl an biometrischen Datenparametern, die insbesondere eine Kombination verschiedener Kategorien von biometrischen Daten einschließt, die zur Ermittlung des mindestens einen aktuellen Belastungsniveaus herangezogen werden, erlaubt die in dieser Anmeldung offenbarte Vorrichtung sowie das offenbarte Verfahren und die offenbarte Applikation eine sehr viel zuverlässigere Ermittlung des mindestens einen aktuellen Belastungsniveaus als es die eingangs erwähnten, bekannten Vorrichtungen und Verfahren bieten, die ein aktuelles Belastungsniveau nur auf der Basis eines einzigen oder sehr weniger biometrischer Datenparameter derselben Kategorie ermitteln.

Darüber hinaus wird die Qualität der Analyse der biometrischen Daten weiter durch das verwendete neuronale Netze Verfahren erhöht, da es mit fortschreitender Zeit und größer werdender Datenbasis, die zum Training des Neuronalen Netzes zur Verfügung steht, immer genauere Aussagen machen kann und somit die Zuverlässigkeit des Verfahrens zur Bestimmung eines aktuellen Belastungsniveaus weiter verbessert.

Wie die genannten Anwendungsfälle zeigen, kann die weitere Applikation eine Mehrzahl von biometrischen Daten zum Benutzer, die zu verschiedenen Kategorien gehören, allein aus den benutzerspezifischen Nutzungsdaten von verfügbaren Applikationen des mobilen Endgerätes einerseits und aus Signaldaten andererseits, die in das mobile Endgerät integrierte Sensoren erzeugen, bestimmen. Somit bietet die erfindungsgemäße Vorrichtung und das erfindungsgemäße Verfahren und die erfindungsgemäße Applikation einem Benutzer eine kostengünstige und zeitunaufwändige Lösung zur Bestimmung eines aktuellen Belastungsniveaus an. Darüber hinaus verzichtet die erfindungsgemäße Lösung auf zusätzliche Vorrichtungen, insbesondere Sensoren, die der Benutzer direkt am Körper fixieren oder tragen muss. Die Lösung schränkt den Benutzer hinsichtlich Komfort, Wohlbefinden oder Optik in keiner Weise ein, wie es das Aufbringen oder Tragen spezifischer Vorrichtungen mit Sensoren mit sich bringt.

Das mindestens eine von der Auswertungseinheit bestimmte aktuelle Belastungsniveau kann dem Benutzer entweder über das Internet zugänglich gemacht werden oder es kann dem Benutzer auf dem mobilen Endgerät zugänglich gemacht werden; beispielsweise durch Versendung einer SMS an den Benutzer. Die Analysedaten bestehend aus dem mindestens einen aktuellen Belastungsniveau und ggf. weiteren Auswertungsdaten, etwa Statistiken zur zeitlichen Änderung eines Belastungsniveaus, können aber auch unter Nutzung derselben Übertragungswege wie bei der Übertragung von biometrischen Daten vom mobilen Endgerät an den zentralen Server, allerdings in umgekehrter Richtung, an das mobile Endgerät übertragen werden. Dazu ist auf dem zentralen Server eine Übertragungseinheit zur Übertragung von Daten von dem Server an das mobile Endgerät vorgesehen. Ebenso ist auf dem mobilen Endgerät eine Empfangseinheit zum Empfang von Daten von dem zentralen Server vorgesehen. Die Analysedaten können auch über einen Push-Service von dem zentralen Server an das mobile Endgerät übertragen werden. Die Datenübertragung erfolgt wiederum verschlüsselt.

Weitere Einzelheiten und Vorteile der Erfindung werden bei der nachfolgenden Beschreibung von Ausführungsbeispielen anhand der Figuren deutlich. Es zeigen:
- Figur 1: eine schematische Darstellung der Vorrichtung zur Ermittlung eines aktuellen Belastungsniveaus;
- Figur 2: eine schematische Darstellung der weiteren Applikation zur Ermittlung eines aktuellen Belastungsniveaus;
- Figur 3a: ein Ablaufschema eines ersten Anwendungsfalls Schlaf;
- Figur 3b: ein Ablaufschema eines zweiten Anwendungsfalls Motorik;
- Figur 3c: ein Ablaufschema eines dritten Anwendungsfalls Sprache;
- Figur 4a: eine grafische Benutzeroberfläche zum Start des Anwendungsfalls Schlaf;
- Figur 4b: eine weitere grafische Benutzeroberfläche einer ersten Auswertungsanzeige;
- Figur 4c: eine weitere grafische Benutzeroberfläche einer zweiten Auswertungsanzeige;
- Figur 4d: eine weitere grafische Benutzeroberfläche einer dritten Auswertungsanzeige;
- Figur 5a: eine schematische Darstellung einer beispielhaften Ausführungsform der Auswertungseinheit;
- Figur 5b: eine schematische Darstellung einer alternativen beispielhaften Ausführungsform der Auswertungseinheit;
- Figur 5c: eine schematische Darstellung einer weiteren alternativen beispielhaften Ausführungsform der Auswertungseinheit.
- Figur 6: eine schematische Darstellung einer beispielhaften Ausführungsform der Auswertungseinheit mit einer Mehrzahl von künstlichen neuronalen Netzen

### Beschreibung von Ausführungsbeispielen der Erfindung

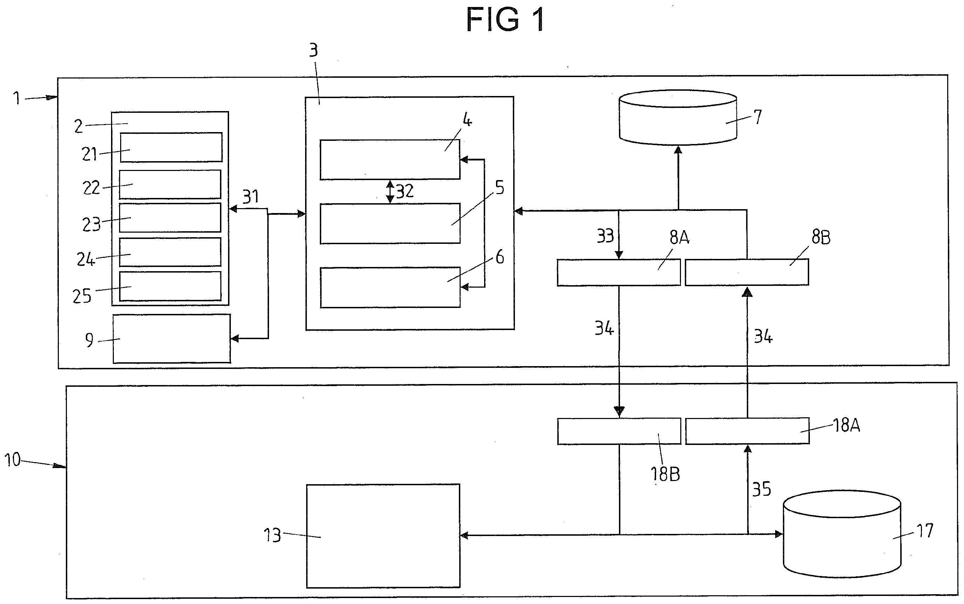

Figur 1 zeigt eine schematische Darstellung einer Ausführungsform der Vorrichtung zur Ermittlung eines aktuellen Belastungsniveaus 36, 36A, 36B, 36C, 36D. Die Vorrichtung umfasst ein mobiles Endgerät 1 und einen zentralen Server 10.

Im mobilen Endgerät 1 sind eine Mehrzahl von Sensoren 2 angeordnet, beispielsweise ein Gyroskop 21, ein Beschleunigungssensor 22, ein Lichtsensor 23 und/oder ein Mikrofon 24. Die von den Sensoren 2 erzeugten Signaldaten 31 sind über ein Betriebssystem 4 zugreifbar. Das Betriebssystem 4 wird innerhalb einer Ausführungseinheit 3 ausgeführt und verwaltet den Zugriff auf die Hardwarekomponenten des mobilen Endgerätes 1, beispielsweise die Sensoren 2. Daneben werden verschiedene Applikationen, beispielsweise eine Mehrzahl von verfügbaren Applikationen 5 und eine weitere Applikation 6, in der Ausführungseinheit 3 ausgeführt.

Die weitere Applikation 6 ermittelt eine Mehrzahl biometrischer Daten 33 zu einem Benutzer des mobilen Endgeräts 1. Die weitere Applikation 6 ist beispielsweise in der Programmiersprache Java implementiert. Die weitere Applikation 6 verwendet als Basis-Entwurfsmuster das MVC(Model-View-Controller)-Entwurfsmuster. Durch die Verwendung des MVC-Entwurfsmusters wird die weitere Applikation 6 derart strukturiert, dass dadurch die Verständlichkeit sowie die Erweiterbarkeit und Anpassbarkeit der weiteren Applikation 6 an neue und/oder veränderte Hardwarekomponenten und Betriebssysteme 4 erleichtert wird.

Die weitere Applikation 6 gewinnt die biometrischen Daten 33 aus Signaldaten 31, die von den Sensoren 2 erzeugt werden und auf die es mittels des Betriebssystems 4 zugreifen kann. Der Zugriff auf die Signaldaten 31 wird von der weiteren Applikation 6 beispielsweise durch Benutzung des Beobachter-Entwurfsmusters realisiert. Das Beobachter-Entwurfsmuster bietet der weiteren Applikation 6 einen vereinfachten und vereinheitlichten Zugriff auf die Signaldaten 31.

Die weitere Applikation 6 kann eine Mehrzahl weiterer biometrischer Daten 33 auch aus den Nutzungsdaten 32 von verfügbaren Applikationen 5 extrahieren. Die von den verfügbaren Applikationen 5 erzeugten Nutzungsdaten 32 sind über das Betriebssystem 4 zugreifbar. Der Zugriff auf die Nutzungsdaten 32 wird von der weiteren Applikation 6 beispielsweise durch Benutzung des Beobachter-Entwurfsmusters realisiert. Das Beobachter-Entwurfsmuster bietet der weiteren Applikation 6 einen vereinfachten und vereinheitlichten Zugriff auf die Nutzungsdaten 32. Ein Beobachter wird über Statusänderungen an dem von ihm beobachteten Objekt, beispielsweise einer verfügbaren Applikation 5, informiert. Handelt es sich bei der verfügbaren Applikation 5 beispielsweise um eine SMS-Applikation und ruft der Benutzer die SMS-Applikation auf, um eine neue SMS zu schreiben, dann wird der Beobachter, der die SMS-Applikation beobachtet, über diese Statusänderung informiert. Die weitere Applikation 6 reagiert auf die vom Beobachter beobachtete Anlage einer neuen SMS, indem die vom Benutzer, beispielsweise über eine Tastatur, eingegebenen Zeichen aufgezeichnet, mit Zeitstempel versehen und als Nutzungsdaten 32 der SMS-Applikation in der lokalen Speichereinheit 7 gespeichert werden.

Es können beispielsweise auch alle Tastatureingaben des Benutzers unabhängig von ihrer Verwendung in einer spezifischen Applikation aufgezeichnet werden. Dazu wird ein Beobachter oder eine Mehrzahl von Beobachtern für den Sensor Tastatur 25 implementiert, beispielsweise ein Beobachter für jede Taste der Tastatur. Sobald eine Taste der Tastatur durch den Benutzer gedrückt wird, wird der die Taste beobachtende Beobachter von diesem Tastendruck informiert. Die weitere Applikation 6 reagiert auf das vom diesem Beobachter beobachtete Drücken der Taste, indem die weitere Applikation 6 prüft, ob der Benutzer eine Löschtaste oder eine andere Taste gedrückt hat. Die Information Löschtaste bzw. andere Taste wird durch die weitere Applikation 6 aufgezeichnet, mit einem Zeitstempel versehen, und diese Daten werden als Signaldaten 31 in der lokalen Speichereinheit 7 gespeichert.

Aus den gespeicherten Signaldaten 31 und/oder Nutzungsdaten 32 extrahiert die weitere Applikation 6 eine Mehrzahl von biometrischen Daten 33. Die biometrischen Daten 33 sind in Kategorien gegliedert, beispielsweise in die Kategorien Schlaf, Sprache, Motorik, Soziale Interaktion, Ökonomische Daten, persönliche Daten und Fragebogen-Daten. Zu jeder Kategorie ist ein kategoriespezifisches Ermittlungs-Zeitintervall definiert, beispielsweise 30 Sekunden für die Kategorie Schlaf und 20 Millisekunden für die Kategorie Sprache. Die für eine Kategorie relevanten Signaldaten 31 und/oder Nutzungsdaten 32 werden in einem ersten Vorverarbeitungsschritt über kategoriespezifische Zeitintervalle zu aufbereiteten Signaldaten 31A und/oder aufbereiteten Nutzungsdaten 32A verarbeitet. Zur Bestimmung der für ein Erfassungs-Zeitintervall relevanten Daten werden die zu den Signaldaten 31 und/oder Nutzungsdaten 32 gespeicherten Zeitstempel ausgewertet. Die aufbereiteten Signaldaten 31A und/oder aufbereiteten Nutzungsdaten 32A werden wiederum mit einem Zeitstempel versehen. In einem zweiten Verarbeitungsschritt werden aus einer Folge von aufbereiteten Signaldaten 31A und/oder aufbereiteten Nutzungsdaten 32A die biometrischen Daten 33 extrahiert. Beispielsweise werden zu einem Anwendungsfall, beispielsweise dem Schreiben einer SMS, aus den aufbereiteten Nutzungsdaten 32A zu der geschriebenen SMS biometrische Daten 33 der Kategorie Motorik ermittelt. Die in einem Anwendungsfall ermittelten biometrischen Daten 33 zu einer Kategorie werden auch als Merkmalsvektor zu dieser Kategorie bezeichnet. Zu jedem Merkmalsvektor wird ein Zeitstempel bestimmt, der das Zeitintervall festlegt, für das der Merkmalsvektor gültig ist. Die biometrischen Daten 33 umfassen die zu den verschiedenen Kategorien ermittelten Merkmalsvektoren mit ihren jeweiligen Zeitstempeln. Die von der weiteren Applikation 6 ermittelten biometrischen Daten 33 werden in einer lokalen Speichereinheit 7 des mobilen Endgerätes 1 gespeichert.

Des Weiteren verfügt das mobile Endgerät 1 über eine Übertragungseinheit 8A und eine Empfangseinheit 8B. Die Übertragungseinheit 8A überträgt Daten 34 von dem mobilen Endgerät 1 an einen externen Knoten, beispielsweise den zentralen Server 10. Die Übertragung erfolgt beispielsweise über die Luftschnittstelle. Die Empfangseinheit 8B empfängt Daten von einem externen Knoten, beispielsweise dem zentralen Server 10. Über die Übertragungseinheit 8A werden Daten 34, beispielsweise die biometrischen Daten 33 des Benutzers, zur Auswertung an den zentralen Server 10 übertragen. Über die Empfangseinheit 8B werden von dem zentralen Server 10 stammende Daten 34, beispielsweise von dem zentralen Server erstellte Auswertungen 35, empfangen. Jede Auswertung 35 ist mit einem Zeitstempel versehen, der das Zeitintervall festlegt, für das die Auswertung gültig ist. Eine Auswertung 35, etwa ein aktuelles Belastungsniveau 36, 36A, 36B, 36C, 36D eines Benutzers des mobilen Endgerätes 1, wird der weiteren Applikation 6 zur Anzeige übergeben und dem Benutzer mittels des Betriebssystems 4 auf dem Display 9 des mobilen Endgerätes 1 angezeigt.

Der zentrale Server 10 verfügt über eine Übertragungseinheit 18A und eine Empfangseinheit 18B. Über die Empfangseinheit 18B werden Daten 34 von einem anderen Knoten, etwa dem mobilen Endgerät 1, empfangen. Bei den empfangenen Daten 34 handelt es sich beispielsweise um biometrische Daten 33 des Benutzers des mobilen Endgerätes 1. Die empfangenen Daten 34 werden in einer zentralen Speichereinheit 17 gespeichert. Des Weiteren ist eine Auswertungseinheit 13 auf dem zentralen Server 10 vorgesehen. Die Auswertungseinheit 13 wertet die empfangenen biometrischen Daten 33 aus. Beispielsweise bestimmt die Auswertungseinheit 13 das mindestens eine aktuelle Belastungsniveau 36, 36A, 36B, 36C, 36D zu einem Zeitpunkt t, indem diejenigen Merkmalsvektoren der empfangenen biometrischen Daten 33 ausgewertet werden, deren Zeitstempel zum Zeitpunkt t gültig sind.

Das aktuelle Belastungsniveaus 36A bestimmt ein erstes aktuelles Belastungsniveau des Benutzers hinsichtlich einer ersten Kategorie von biometrischen Daten 33, beispielsweise der Kategorie Schlaf. Das aktuelle Belastungsniveaus 36C bestimmt ein zweites aktuelles Belastungsniveau des Benutzers hinsichtlich einer zweiten Kategorie von biometrischen Daten 33, beispielsweise der Kategorie Motorik. Das aktuelle Belastungsniveaus 36B bestimmt ein drittes aktuelles Belastungsniveau des Benutzers hinsichtlich einer dritten Kategorie von biometrischen Daten 33, beispielsweise der Kategorie Sprache. Das aktuelle Belastungsniveaus 36D bestimmt ein viertes aktuelles Belastungsniveau des Benutzers hinsichtlich einer vierten Kategorie von biometrischen Daten 33, beispielsweise der Kategorie Soziale Interaktion, oder hinsichtlich einer Kombination von Kategorien von biometrischen Daten, beispielsweise der Kategorien Soziale Interaktion, Ökonomische Daten, Persönliche Daten und/oder Fragebogen-Daten. Daneben können weitere aktuelle Belastungsniveaus hinsichtlich weiterer Kategorien und/oder Kombinationen von Kategorien bestimmt werden. Das aktuelle Belastungsniveau 36 bestimmt ein konsolidiertes aktuelles Belastungsniveau des Benutzers, welches sich aus einer Kombination der kategoriespezifischen Belastungsniveaus 36A, 36B, 36C, 36D und gegebenenfalls vorliegender, weiterer kategoriespezifischer Belastungsniveaus ergibt, beispielsweise durch Bildung des arithmetischen Mittels der kategoriespezifischen Belastungsniveaus.

Die von der Auswertungseinheit 13 bestimmte mindestens eine Auswertung 35, etwa das mindestens eine aktuelle Belastungsniveau 36, 36A, 36B, 36C, 36D, umfasst für jede Auswertung 35 einen Zeitstempel, der das Zeitintervall festlegt, für das die Auswertung 35 gültig ist. Die mindestens eine Auswertung 35, etwa das mindestens eine aktuelle Belastungsniveau 36, 36A, 36B, 36C, 36D wird in der zentralen Speichereinheit 17 gespeichert und über die Übertragungseinheit 18A an das mobile Endgerät 1 übertragen.

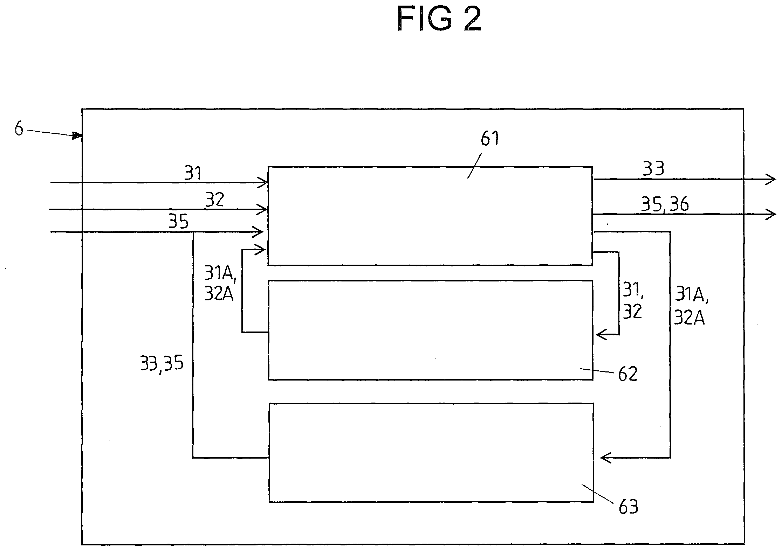

Figur 2 zeigt eine schematische Darstellung einer Ausführungsform der weiteren Applikation 6 zur Ermittlung mindestens eines aktuellen Belastungsniveaus 36, 36A, 36B, 36C, 36D. Die weitere Applikation 6 umfasst eine Mehrzahl von Komponenten, etwa einen Datenmanager 61, einen Datenpräprozessor 62 sowie einen Datenanalysator 63.

Die von dem Betriebssystem 4 verfügbar gemachten Signaldaten 31 und/oder Nutzungsdaten 32 werden in den Datenmanager 61 geladen und von diesem verwaltet. Der Datenmanager 61 übergibt die Signaldaten 31 und/oder Nutzungsdaten 32 an den Datenpräprozessor 62. Der Datenpräprozessor 62 bereitet die Signaldaten 31 und/oder Nutzungsdaten 32 auf und übergibt die aufbereiteten Signaldaten 31A und/oder aufbereiteten Nutzungsdaten 32A zurück an den Datenmanager 61. Der Datenmanager 61 speichert die aufbereiteten Signaldaten 31A und/oder aufbereiteten Nutzungsdaten 32A in der lokalen Speichereinheit 7. Der Datenmanager 61 übergibt die aufbereiteten Signaldaten 31A und/oder aufbereiteten Nutzungsdaten 32A an den Datenanalysator 63. Der Datenanalysator 63 analysiert die aufbereiteten Signaldaten 31A und/oder aufbereiteten Nutzungsdaten 32A und bestimmt daraus die biometrischen Daten 33. Für diejenigen biometrischen Daten 33, die lokal ausgewertet werden, erstellt der Datenanalysator 63 mindestens eine Auswertung 35, beispielsweise in Form von mindestens einem aktuellen Belastungsniveaus 36, 36A, 36B, 36C, 36D. Der Datenanalysator 63 übergibt die biometrischen Daten 33 und gegebenenfalls die mindestens eine Auswertung 35 an den Datenmanager 61. Soweit mindestens eine Auswertung 35 durch den Datenanalysator 63 erstellt wurde, visualisiert der Datenmanager 61 die mindestens eine Auswertung 35 für den Benutzer des mobilen Endgerätes 1 durch Anzeige auf dem Display 9. Der Datenmanager 61 übergibt die biometrischen Daten 33 an die Übertragungseinheit 8A zur Übertragung an den zentralen Server 10, soweit die biometrischen Daten 33 zentral ausgewertet werden.

Diejenige Auswertung 35, die in Form des konsolidierten aktuellen Belastungsniveaus 36 gegeben ist, kann beispielsweise kontinuierlich als Ampel-Icon auf dem Display 9 visualisiert werden. Das Ampel-Icon kann in Abhängigkeit von dem konsolidierten aktuellen Belastungsniveau 36 die Farben grün, gelb oder rot anzeigen. Wird das konsolidierte aktuelle Belastungsniveau 36 etwa auf einen ganzzahligen Wert im Wertebereich [0,10] normiert, so wird die Ampelfarbe in Abhängigkeit vom aktuellen Wert des konsolidierten aktuellen Belastungsniveaus 36 gewählt. Ein hoher Wert entspricht einem hohen konsolidierten aktuellen Belastungsniveau 36. Ein niedriger Wert entspricht einem niedrigen konsolidierten aktuellen Belastungsniveau 36. Ist das konsolidierte aktuelle Belastungsniveau 36 niedrig, etwa im Wertebereich [0,3], so wird die Farbe Grün angezeigt. Ist das konsolidierte aktuelle Belastungsniveau 36 erhöht, etwa im Wertebereich [4,6], so wird die Farbe Gelb angezeigt. Ist das konsolidierte aktuelle Belastungsniveau 36 des Benutzers hoch, etwa im Wertebereich [7,10], so wird die Farbe Rot angezeigt. Die Anzeige des konsolidierten aktuellen Belastungsniveaus 36 wird aktualisiert sobald ein konsolidiertes aktuelles Belastungsniveau 36 mit einem Zeitstempel vorliegt, der jünger ist, als der Zeitstempel des bisher angezeigten konsolidierten Belastungsniveaus.

Zum Beispiel, wird das konsolidierte aktuelle Belastungsniveau 36 als Balkendiagramm mit 10 Balken visualisiert. Jedem Balken im Balkendiagramm ist ein ganzzahliger Wert aus dem Wertebereich [0,10], auf das das konsolidierte aktuelle Belastungsniveau 36 normiert ist, zugeordnet.

Falls biometrische Daten 33 zur Auswertung an den zentralen Server 10 übergeben wurden, erhält der Datenmanager 61 mindestens eine Auswertung 35, beispielsweise in Form eines dritten aktuellen Belastungsniveaus 36B zur Kategorie Sprache, zu den serverseitig ausgewerteten biometrischen Daten 33 zur Kategorie Sprache.

Wenn der Datenmanager 61 eine neue Auswertung 35 erhält, beispielsweise in Form eines dritten aktuellen Belastungsniveaus 36B zur Kategorie Sprache, so ermittelt er ein neues konsolidiertes aktuelles Belastungsniveau 36 aus den dem Datenmanager 61 bekannten kategoriespezifischen aktuellen Belastungsniveaus, deren Zeitstempel aktuell noch gültig sind. Das konsolidierte aktuelle Belastungsniveau 36 ergibt sich beispielsweise durch das arithmetische Mittel oder durch ein gewichtetes Mittel der noch gültigen kategoriespezifischen aktuellen Belastungsniveaus 36A, 36B, 36C, 36D. Der Datenmanager 61 visualisiert das konsolidierte aktuelle Belastungsniveau 36 auf dem Display 9, beispielsweise durch Aktualisierung des Ampel-Icons.

Bei dem konsolidierten aktuellen Belastungsniveau 36 des Benutzers handelt es sich um eine individuelle Größe. Beispielsweise kann bei einer ersten Benutzung der weiteren Applikation 6 durch einen Benutzer eine benutzerspezifische Kalibrierung vorgenommen werden. Dazu wird der Benutzer aufgefordert biometrische Daten 33 der Kategorie Persönliche Daten zu erfassen, beispielsweise über ein in die weitere Applikation 6 integriertes Formular. Auf Basis der Persönlichen Daten wird ein individuelles aktuelles Belastungsniveau des Benutzers bestimmt, welches beispielsweise einen Kalibrierungsfaktor festlegt. Das individuelle aktuelle Belastungsniveau, beispielsweise in seiner Ausprägung als Kalibrierungsfaktor, wird bei der Bestimmung des aktuellen Belastungsniveaus 36, 36A, 36B, 36C, 36D zum Benutzer berücksichtigt.

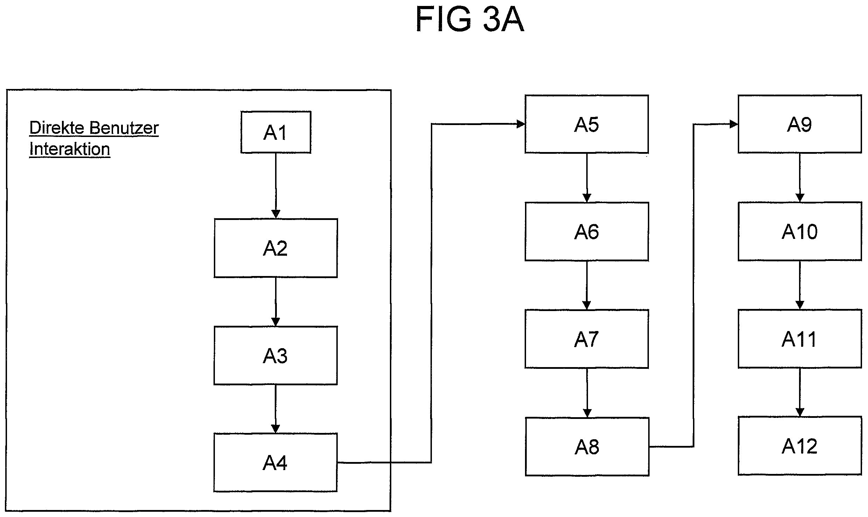

Figur 3A zeigt ein Ablaufschema eines ersten Anwendungsfalls Schlaf. Der erste Anwendungsfall Schlaf ermittelt biometrische Daten 33 der Kategorie Schlaf zur Ermittlung eines ersten aktuellen Belastungsniveaus 36A eines Benutzers. Der erste Anwendungsfall beschreibt ein erstes Verfahren zur Ermittlung dieses ersten aktuellen Belastungsniveaus 36A. Vor einer ersten Verwendung des Anwendungsfalls Schlaf lässt der Benutzer das mobile Endgerät 1 aus circa 30 Zentimetern Höhe auf seine Matratze fallen. Durch Auswertung der Sensordaten 2 berechnet die weitere Applikation 6 die Federhärte und die Dämpfungskonstante der Matratze, welche als Kalibrierungsdaten zum Anwendungsfall Schlaf gespeichert werden. Der Anwendungsfall Schlaf ermittelt Bewegungsdaten während der Ruhephase des Benutzers und wertet diese aus.
(A1): Für den Anwendungsfall Schlaf bedarf es einer direkten Benutzer-Interaktion.
(A2): Dazu ruft der Benutzer des mobilen Endgerätes 1 den Schlafmodus der weiteren Applikation 6 auf. In einer möglichen Ausführungsform wird bei Aufruf des Schlafmodus das mobile Endgerät 1 automatisch in den Flugmodus versetzt, um Emissionen von elektromagnetischer Strahlung durch das mobile Endgerät 1 zu minimieren. Der Benutzer positioniert das mobile Endgerät 1 während seiner Ruhephase auf der Matratze.
(A3): Die während der Ruhephase von den Sensoren 2, beispielsweise dem Gyroskop 21, dem Beschleunigungssensor 22 und dem Lichtsensor 23, produzierten Signaldaten 31 werden durch die weitere Applikation 6 gesammelt und in der lokalen Speichereinheit 7 gespeichert.
(A4): Nach Beenden der Ruhephase deaktiviert der Benutzer den Schlafmodus in der weiteren Applikation 6. Gegebenenfalls wird dadurch auch der Flugmodus deaktiviert und damit die Übertragungseinheit 8A und die Empfangseinheit 8B aktiviert.
(A5): Der Datenmanager 61 der weiteren Applikation 6 lädt die während des Schlafmodus ermittelten Sensordaten in die weitere Applikation 6 und übergibt diese Signaldaten 31 an den Datenpräprozessor 62.
(A6): Der Datenpräprozessor 62 teilt die ermittelten Signaldaten 31 in Zeitintervalle, beispielsweise in Zeitintervalle der Länge 30 Sekunden. Zu den Signaldaten 31 jedes Zeitintervalls werden für das Zeitintervall charakteristische aufbereitete Signaldaten 31A bestimmt, die mit einem Zeitstempel versehen werden.
(A7): Der Datenpräprozessor 62 übergibt die aufbereiteten Signaldaten 31A mit ihren Zeitstempeln an den Datenmanager 61.
(A8): Der Datenmanager 61 speichert die aufbereiteten Signaldaten 31A mit ihren Zeitstempeln in der lokalen Speichereinheit 7.
(A9): Der Datenmanager 61 übergibt die aufbereiteten Signaldaten 31A mit ihren Zeitstempeln zur Auswertung an den Datenanalysator 63.
(A10): Der Datenanalysator 63 analysiert die aufbereiteten Signaldaten 31A und bestimmt aus ihnen einen Merkmalsvektor mit biometrischen Daten 31 der Kategorie Schlaf. Die Bestimmung des Merkmalsvektors geschieht beispielsweise mittels eines statistischen Regressionsmodells zur Modellierung einer binären Zielvariablen, beispielsweise eines Logit- oder Probit-Modells. Hierzu wird die Folge der aufbereiteten Signaldaten 31A, die sich durch Anordnung der aufbereiteten Signaldaten 31A nach aufsteigenden Zeitstempeln ergibt, ausgewertet, und jedes Folgenglied wird hinsichtlich des Schlafzustands als "Wach" oder "Schlaf" klassifiziert. Für die Klassifizierung werden die Schlafzustände der vorangegangenen Folgenglieder, also die Schlafzustände in den vorangegangenen Zeitintervallen, berücksichtigt. Ist die Wahrscheinlichkeit, dass sich der Benutzer in einem Zeitintervall in schlafendem Zustand befindet, größer als 50%, so wird das Zeitintervall mit dem Zustand "Schlaf" klassifiziert, andernfalls mit dem Zustand "Wach". Auf Basis der Folge der Schlafzustände über alle Zeitintervalle wird der Merkmalsvektor bestimmt.

Der Merkmalsvektor der biometrischen Daten 33 zur Kategorie Schlaf umfasst beispielsweise die folgenden Merkmale:
a. Einschlaflatenz
b. Schlafeffizienz
c. Einschlafzeitpunkt
d. Schlafende
e. Zeit im Bett
f. Schlafdauer
g. Wachzeit
h. Dauer bis zur ersten REM-Phase
i. Stadienanteile der einzelnen Schlafphasen
j. Anzahl Erwachen
k. Anzahl Schlafstadienwechsel

Der Datenanalysator 63 bestimmt aus dem Merkmalsvektor der biometrischen Daten 33 zur Kategorie Schlaf eine Auswertung 35, die insbesondere das erste aktuelle Belastungsniveau 36A zur Kategorie Schlaf umfasst. Zur Ermittlung des ersten aktuellen Belastungsniveaus 36A werden beispielsweise alle Merkmale des Merkmalsvektors mit einem ganzzahligen Wert aus dem Wertebereich [0,10] bewertet und aus den Einzelwerten wird ein Mittelwert gebildet, beispielsweise ein arithmetisches Mittel oder ein gewichtetes Mittel. Zusätzlich fließt in die Bewertung die benutzerspezifische Kalibrierung, beispielsweise durch einen zu berücksichtigenden Kalibrierungsfaktor ein. Das erste aktuelle Belastungsniveau 36A zur Kategorie Schlaf ergibt sich beispielsweise als ganzzahliger Wert im Wertebereich [0,10]. Das erste aktuelle Belastungsniveau 36A umfasst einen Zeitstempel, der den Zeitraum festlegt, zu dem das erste aktuelle Belastungsniveau 36A zur Kategorie Schlaf gültig ist.
(A11): Der Merkmalsvektor der biometrischen Daten 33 zur Kategorie Schlaf sowie die Auswertung 35, die insbesondere das erste aktuelle Belastungsniveau 36A zur Kategorie Schlaf umfasst, werden an den Datenmanager 61 übergeben.
(A12): Der Datenmanager 61 speichert den Merkmalsvektor der biometrischen Daten 33 zur Kategorie Schlaf sowie die Auswertung 35, insbesondere das erste aktuelle Belastungsniveau 36A zur Kategorie Schlaf in der lokalen Speichereinheit 7. Der Datenmanager 61 visualisiert die Auswertung 35, insbesondere das erste aktuelle Belastungsniveau 36A zur Kategorie Schlaf, auf dem Display 9. Aus dem ersten aktuellen Belastungsniveau 36A zur Kategorie Schlaf und gegebenenfalls weiteren vorliegenden, gültigen aktuellen Belastungsniveaus zu weiteren Kategorien, beispielsweise den aktuellen Belastungsniveaus 36B, 36C, 36D, bestimmt der Datenmanager 61 ein konsolidiertes aktuelles Belastungsniveau 36 und visualisiert das konsolidierte aktuelle Belastungsniveau 36, beispielsweise durch Aktualisierung des Ampel-Icons.

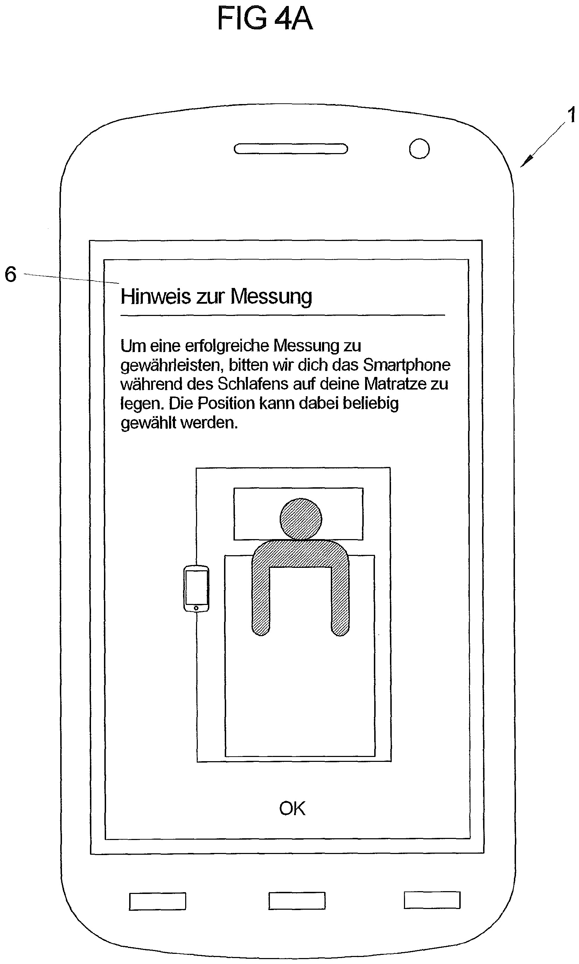

Figur 4A zeigt eine exemplarische grafische Benutzeroberfläche zum Start des Anwendungsfalls Schlaf der weiteren Applikation 6. Die exemplarische grafische Benutzeroberfläche enthält einen Hinweis zur erfolgreichen Messung der biometrischen Daten 33 zur Kategorie Schlaf. Durch Auswahl der OK-Schaltfläche kann der Benutzer den Anwendungsfall starten.

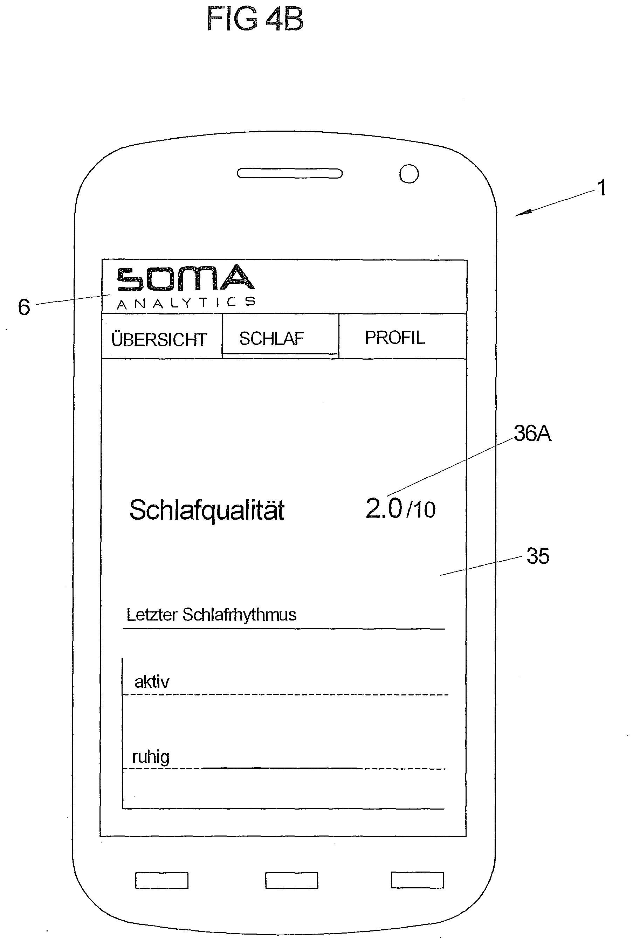

Figur 4B zeigt eine weitere beispielhafte grafische Benutzeroberfläche einer ersten Auswertungsanzeige zum Anwendungsfall Schlaf. Die erste Auswertungsanzeige visualisiert eine Auswertung 35 zur Kategorie Schlaf in der Ausprägung einer Übersichtsauswertung. Mit dem Parameter Schlafqualität wird ein erstes aktuelles Belastungsniveau 36A des Benutzers zur Kategorie Schlaf angezeigt. Die Schlafqualität wird mit dem numerischen Wert 2.0 innerhalb einer Skala von 0 bis 10 angegeben. Daneben umfasst die erste Auswertungsanzeige weitere Elemente, beispielsweise den letzten Schlafrhythmus als Funktion von der Zeit.

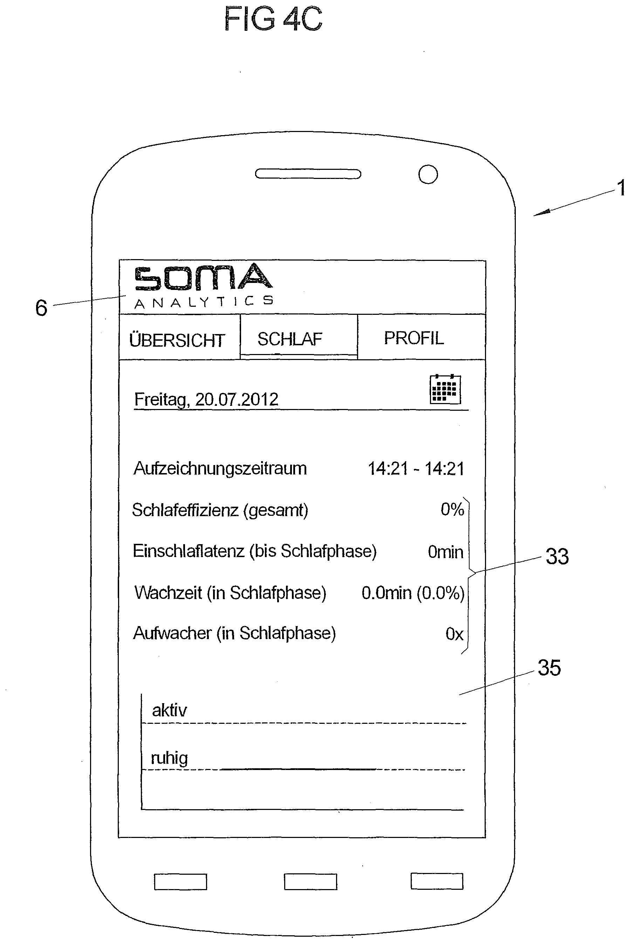

Figur 4C zeigt eine weitere exemplarische grafische Benutzeroberfläche einer zweiten Auswertungsanzeige zum Anwendungsfall Schlaf. Die zweite Auswertungsanzeige visualisiert eine Auswertung 35 zur Kategorie Schlaf in der Ausprägung einer Detailanzeige. Die Detailanzeige umfasst die ermittelten biometrischen Daten 33 zur Kategorie Schlaf. Zu jedem Merkmal der biometrischen Daten 33 der Kategorie Schlaf wird der ermittelte Wert angegeben.

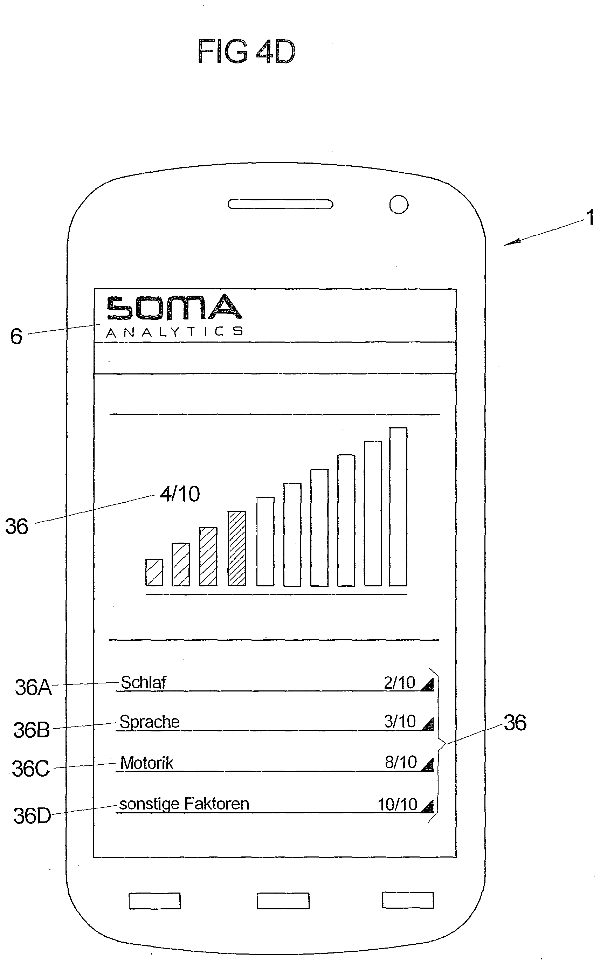

Figur 4D zeigt eine weitere grafische Benutzeroberfläche einer dritten Auswertungsanzeige zum Anwendungsfall Schlaf. Die dritte Auswertungsanzeige visualisiert das konsolidierte aktuelle Belastungsniveau 36 in einem Balkendiagramm. Zusätzlich werden das konsolidierte Belastungsniveau 36 und die aktuellen Belastungsniveaus 36A, 36B, 36C, 36D zu den einzelnen Kategorien als numerische Werte angezeigt. Jeder numerische Wert wird in einer für den Wert spezifischen Farbe angezeigt. Durch die Farbwahl werden die aktuellen Belastungsniveaus 36, 36A, 36B, 36C, 36D farblich visualisiert.

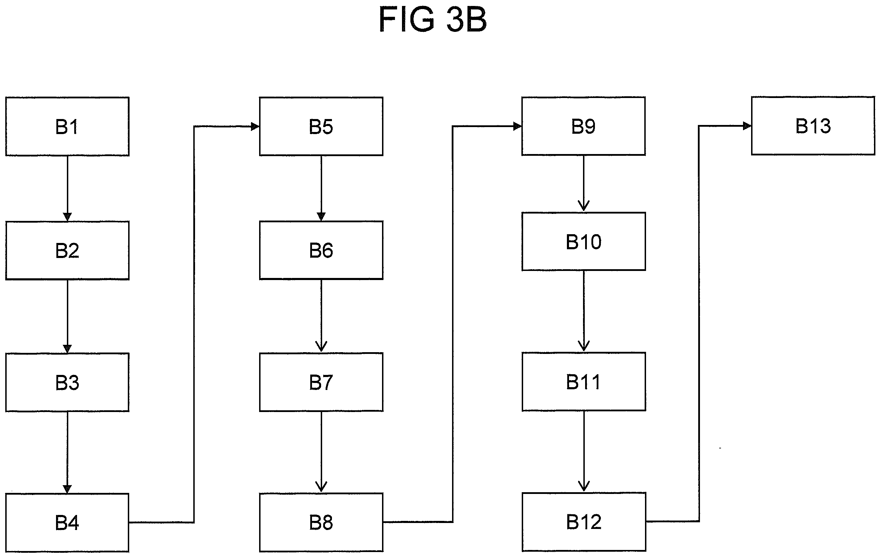

Figur 3B zeigt ein Ablaufschema eines zweiten Anwendungsfalls Motorik. Der zweite Anwendungsfall Motorik ermittelt biometrische Daten 33 der Kategorie Motorik zur Ermittlung eines zweiten aktuellen Belastungsniveaus 36C eines Benutzers. Der zweite Anwendungsfall beschreibt ein zweites Verfahren zur Ermittlung des zweiten aktuellen Belastungsniveaus 36C. Für diesen Anwendungsfall ist nur eine indirekte Interaktion mit dem Benutzer erforderlich.
(B1): Die weitere Applikation 6 ist in die Ausführungseinheit 3 des mobilen Endgerätes 1 geladen und wurde gestartet. Die weitere Applikation 6 läuft in der Ausführungseinheit 3 als Hintergrundprozess.
(B2): Der Benutzer ruft eine verfügbare Applikation 5 auf, die mit einer Texteingabe über die Tastatur verbunden ist, beispielsweise eine SMS-Applikation zum Erstellen einer neuen SMS.
(B3): Der Benutzer benutzt die Tastatur 25 des mobilen Endgerätes 1, beispielsweise um eine SMS zu tippen.
(B4): Die von dem Benutzer getätigte Folge von Tastatureingaben wird durch den Datenmanager 61 der weiteren Applikation 6 gesammelt und auf der lokalen Speichereinheit 7 gespeichert. Zu jeder Tastatureingabe wird ein Zeitstempel gespeichert.
(B5): Der Benutzer beendet das Tippen, beispielsweise durch Fertigstellung und Versendung der SMS.
(B6): Der Datenmanager 61 übergibt die gesammelten und gespeicherten Tastaturdaten an den Datenpräprozessor 62.
(B7): Der Datenpräprozessor 62 nimmt eine Vorauswertung der Tastaturdaten vor. Dazu teilt der Datenpräprozessor 62 die ermittelten Tastaturdaten in Zeitintervalle, beispielsweise in Zeitintervalle der Länge 15 Sekunden. Zu den Tastaturdaten 32 jedes Zeitintervalls werden für das Zeitintervall charakteristische aufbereitete Nutzungsdaten 32A bestimmt, die mit einem Zeitstempel versehen werden.
(B8): Die aufbereiteten und mit Zeitstempeln versehenen Nutzungsdaten 32A werden von dem Datenpräprozessor 62 an den Datenmanager 61 übergeben.
(B9): Der Datenmanager 61 speichert die aufbereiteten und mit Zeitstempeln versehenen Nutzungsdaten 32A in der lokalen Speichereinheit 7.
(B10): Der Datenmanager 61 übergibt die aufbereiteten und mit Zeitstempeln versehenen Nutzungsdaten 32A an den Datenanalysator 63.
(B11): Der Datenanalysator 63 analysiert die aufbereiteten und mit Zeitstempeln versehenen Nutzungsdaten 32A und bestimmt aus ihnen einen Merkmalsvektor mit biometrischen Daten 31 der Kategorie Motorik.

Beispielsweise bestimmt der Datenanalysator 63 die Fehlerrate aus der Häufigkeit von Tastatur-Fehleingaben, insbesondere aus der Anzahl der Betätigungen einer Löschtaste durch den Benutzer in dem betrachteten Zeitintervall. Die bestimmte Fehlerrate ist ein Maß für die Hand-Auge-Koordination des Benutzers.

Der Merkmalsvektor der biometrischen Daten zur Kategorie Motorik umfasst beispielsweise die folgenden Merkmale:
a. Geschwindigkeit (Anschläge pro Zeiteinheit)
b. Fehlerrate
c. Varianz der Fehlerrate
d. Varianz der Geschwindigkeit

Der Datenanalysator 63 bestimmt aus dem Merkmalsvektor der biometrischen Daten 33 zur Kategorie Motorik eine Auswertung 35, insbesondere das zweite aktuelle Belastungsniveau 36C zur Kategorie Motorik. Dazu werden beispielsweise alle Merkmale des Merkmalsvektors mit einem ganzzahligen Wert aus dem Wertebereich [0,10] bewertet und aus den Einzelwerten wird ein Mittelwert gebildet, beispielsweise ein arithmetisches Mittel oder ein gewichtetes Mittel. Zusätzlich fließt in die Bewertung die benutzerspezifische Kalibrierung ein, beispielsweise durch einen zu berücksichtigenden Kalibrierungsfaktor. Das zweite aktuelle Belastungsniveau 36C zur Kategorie Schlaf ergibt sich beispielsweise als ganzzahliger Wert im Wertebereich [0,10]. Das zweite aktuelle Belastungsniveau 36C umfasst einen Zeitstempel, der den Zeitraum festlegt, zu dem das zweite aktuelle Belastungsniveau 36C zur Kategorie Schlaf gültig ist.
(B12): Der Datenanalysator 63 übergibt den Merkmalsvektor der biometrischen Daten 33 zur Kategorie Schlaf sowie die Auswertung 35, insbesondere das zweite aktuelle Belastungsniveau 36C zur Kategorie Motorik mit seinem Zeitstempel, an den Datenmanager 61.
(B13): Der Datenmanager 61 speichert den Merkmalsvektor der biometrischen Daten 33 zur Kategorie Schlaf sowie die Auswertung 35, insbesondere das zweite aktuelle Belastungsniveau 36C zur Kategorie Motorik mit seinem Zeitstempel, in der lokalen Speichereinheit 7. Der Datenmanager 61 visualisiert die Auswertung 35, insbesondere das zweite aktuelle Belastungsniveau 36C zur Kategorie Motorik, auf dem Display 9. Aus dem zweiten aktuellen Belastungsniveau 36C zur Kategorie Motorik und gegebenenfalls weiteren vorliegenden, gültigen aktuellen Belastungsniveaus zu weiteren Kategorien bestimmt der Datenmanager 61 das konsolidierte aktuelle Belastungsniveau 36 und visualisiert dieses, beispielsweise durch Aktualisierung des Ampel-Icons.

In einer alternativen Ausführungsform werden die biometrischen Daten 31 beispielswiese die biometrischen Daten 31 zu den Kategorien Schlaf und/oder Motorik, an den zentralen Server 10 übertragen, in der zentralen Speichereinheit 17 gespeichert und von der auf dem Server angeordneten Auswertungseinheit 13 ausgewertet.

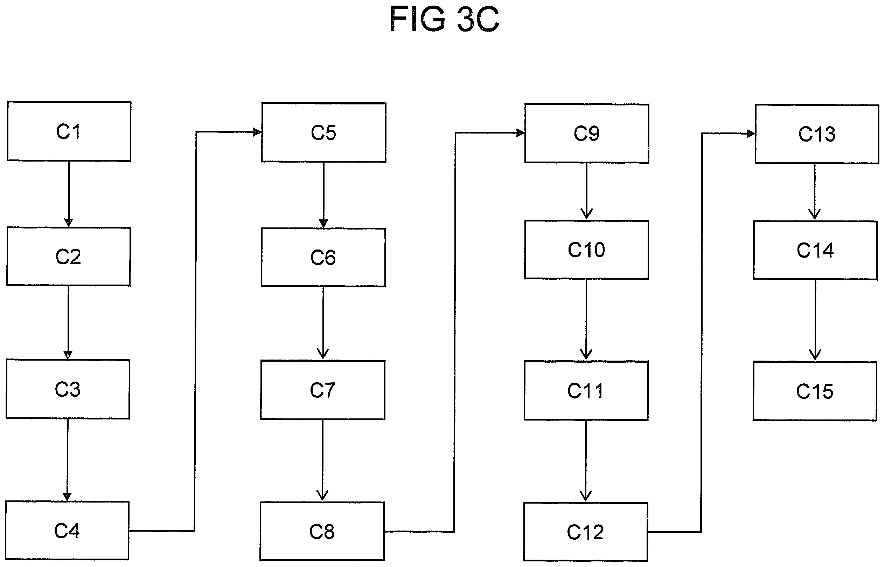

Figur 3C zeigt ein Ablaufschema eines dritten Anwendungsfalls Sprache. Der dritte Anwendungsfall Sprache ermittelt biometrische Daten 33 der Kategorie Sprache, beispielsweise die Sprachparameter Sprechgeschwindigkeit und/oder Modulationsfähigkeit, zur Ermittlung eines dritten aktuellen Belastungsniveaus 36B eines Benutzers. Der dritte Anwendungsfall beschreibt ein drittes Verfahren zur Ermittlung des dritten aktuellen Belastungsniveaus 36B. Für diesen Anwendungsfall ist nur eine indirekte Interaktion mit dem Benutzer erforderlich.

Der Anwendungsfall Sprache umfasst eine Sprachanalyse von Sprachdaten des Benutzers, etwa von Sprachdaten, aus vom Benutzer mit dem mobilen Endgerät 1 geführten Telefonaten.
(C1): Die weitere Applikation 6 ist in die Ausführungseinheit 3 des mobilen Endgerätes 1 geladen und wurde gestartet. Die weitere Applikation 6 läuft in der Ausführungseinheit 3 als Hintergrundprozess.
(C2): Der Anwendungsfall Sprache wird beispielsweise durch einen auf den mobilen Endgerät 1 eingehenden Anruf gestartet.
(C3): Der Benutzer nimmt den Anruf an.
(C4): Der Datenmanager 61 der weiteren Applikation 6 sammelt während des Gespräches kontinuierlich die über das Mikrofon 24 erfassten Sprachdaten 31 zum Benutzer, versieht sie mit einem Zeitstempel und speichert Sprachdaten 31 mit Zeitstempel in der lokalen Speichereinheit 7.
(C5): Der Benutzer beendet das Gespräch.
(C6): Der Datenmanager 61 übergibt die mit Zeitstempel gespeicherten Sprachdaten 31 an den Datenpräprozessor 62.
(C7): Der Datenpräprozessor 62 nimmt eine Vorauswertung der Sprachdaten 31 vor. Dazu teilt der Datenpräprozessor 62 die erfassten Sprachdaten 31 in Zeitintervalle, beispielsweise in Zeitintervalle der Länge 20 Millisekunden. Zu den Sprachdaten 31 jedes Zeitintervalls werden für das Zeitintervall charakteristische aufbereitete Sprachdaten 31A bestimmt, die mit einem Zeitstempel versehen werden.
(C8): Der Datenpräprozessor 62 übergibt die aufbereiteten Sprachdaten 31A mit ihren Zeitstempeln an den Datenmanager 61.
(C9): Der Datenmanager 61 speichert die aufbereiteten Sprachdaten 31A mit ihren Zeitstempeln in der lokalen Speichereinheit 7.
(C10): Der Datenmanager 61 übergibt die aufbereiteten Sprachdaten 31A mit ihren Zeitstempeln zur Auswertung an den Datenanalysator 63.
(C11): Der Datenanalysator 63 analysiert die aufbereiteten Sprachdaten 31A und bestimmt aus ihnen einen Merkmalsvektor mit biometrischen Daten 31 der Kategorie Sprache.

Der Merkmalsvektor der biometrischen Daten 31 zur Kategorie Sprache umfasst beispielsweise die folgenden Merkmale:
a. Akzentausprägung (Accent shape)
b. durchschnittliche Tonhöhe (Average pitch)
c. Konturkurve (Contour slope)
d. Endabsenkung (Final Lowering)
e. Tonhöhenbandweite (Pitch range)
f. Sprechtempo (Speech rate)
g. Beanspruchungsfrequenzbereich (Stress frequency)
h. Rauchigkeit (Breathiness)
i. Stimmbrillianz (Brilliance)
j. Lautstärke (Loudness)
k. Pausendiskontinuität (Pause Discontinuity)
l. Tonhöhendiskontinuität (Pitch Discontinuity)
m. Zeit in verschiedenen Gefühlszuständen (Zustand1,...,Zustandn)

Der Merkmalsvektor wird mit einem Zeitstempel versehen und diese Daten werden als biometrische Daten 33 der Kategorie Sprache von dem Datenanalysator 63 an den Datenmanager 61 übergeben.
(C12): Der Datenmanager 61 speichert den mit einem Zeitstempel versehenen Merkmalsvektor als biometrische Daten 33 der Kategorie Sprache in der lokalen Speichereinheit 7. Der Datenmanager 61 übergibt die biometrischen Daten 33 zur Kategorie Sprache an die Übertragungseinheit 8A zur Übertragung an den zentralen Server 10.
(C13): Die Empfangseinheit 18B des zentralen Servers 10 empfängt die übertragenen Daten in Form der biometrischen Daten 33 zur Kategorie Sprache. Der zentrale Server 10 speichert die biometrischen Daten 33 in der zentralen Speichereinheit 17 und wertet die biometrischen Daten 33 in der Auswertungseinheit 13 aus. Dazu findet beispielsweise ein weiter unten erläutertes neuronale Netze-Verfahren Anwendung. Die Auswertungseinheit 13 bestimmt eine Auswertung 35. Die Auswertung 35 umfasst insbesondere das dritte aktuelle Belastungsniveau 36B der Kategorie Sprache. Das dritte aktuelle Belastungsniveau 36B zur Kategorie Sprache wird beispielsweise als ganzzahliger Wert im Wertebereich [0,10] bestimmt. Das dritte aktuelle Belastungsniveau 36B umfasst einen Zeitstempel, der den Zeitraum festlegt, zu dem das dritte aktuelle Belastungsniveau 36B zur Kategorie Sprache gültig ist.
(C14): Der zentrale Server 10 überträgt die Auswertung 35, insbesondere das dritte aktuelle Belastungsniveau 36B zur Kategorie Sprache mit seinem Zeitstempel, mittels der Übertragungseinheit 18A an das mobile Endgerät 1. Die übertragene Auswertung 35 wird von der Empfangseinheit 8B des mobilen Endgeräts 1 empfangen und an den Datenmanager 61 der weiteren Applikation 6 übergeben.
(C15): Der Datenmanager 61 speichert die Auswertung 35, insbesondere das dritte aktuelle Belastungsniveau 36B zur Kategorie Sprache mit seinem Zeitstempel in der lokalen Speichereinheit 7. Der Datenmanager visualisiert die Auswertung 35, insbesondere das dritte aktuelle Belastungsniveau 36B zur Kategorie Sprache, auf dem Display 9. Aus dem dritten aktuellen Belastungsniveau 36B zur Kategorie Sprache und gegebenenfalls weiteren vorliegenden, gültigen aktuellen Belastungsniveaus zu weiteren Kategorien bestimmt der Datenmanager 61 das konsolidierte aktuelle Belastungsniveau 36 und visualisiert das konsolidierte aktuelle Belastungsniveau 36, beispielsweise durch Aktualisierung des Ampel-Icons.

Neben den genannten Anwendungsfällen Schlaf, Sprache und Motorik gibt es weitere Anwendungsfälle, die weitere biometrische Daten 33 zu weiteren Kategorien ermitteln und daraus weitere aktuelle Belastungsniveaus des Benutzers bestimmen.

So wertet der Anwendungsfall Soziale Interaktion Nutzungsdaten 32 des Benutzers aus solchen verfügbaren Applikationen 5 aus, die der sozialen Interaktion dienen. verfügbare Applikationen 5, die der sozialen Interaktion dienen, sind beispielsweise SMS-Applikationen, E-Mail-Applikationen oder soziale Netzwerk-Applikationen, wie beispielsweise eine Instant Messaging-Applikation oder eine Facebook-Applikation. Aus den Nutzungsdaten 32 zu den verfügbaren Applikationen 5, die der sozialen Interaktion dienen, kann beispielsweise die Anzahl der Kontakte in sozialen Netzwerken oder die Häufigkeit der Kontaktaufnahme, beispielsweise die Häufigkeit der Versendung einer SMS, ermittelt werden.

Der Merkmalsvektor der biometrischen Daten 33 zur Kategorie Soziale Interaktion umfasst beispielsweise die folgenden Merkmale:
a. Anzahl Telefonkontakte
b. Anzahl Kontakte in sozialen Netzwerken
c. Häufigkeit der Kontaktaufnahme (SMS, Telefonieren, Nachrichten im sozialen Netz)
d. Dauer der Kontaktaufnahme
e. Zeitpunkt der Kontaktaufnahme
f. Frequenz der Kontaktaufnahme
g. Absolute und relative Anzahl Kontakte mit regelmäßiger Kontaktaufnahme

Daneben können biometrische Daten 33 weiterer Kategorien berücksichtigt werden, beispielsweise biometrische Daten 33 der Kategorie Ökonomische Daten, der Kategorie Persönliche Daten und/oder der Kategorie Fragebogen-Daten.

Die Kategorie Ökonomische Daten betrifft übergreifende und nicht nutzerspezifische Daten, beispielsweise Daten zum allgemeinen Krankenstand oder zur Arbeitsplatzsicherheit.

Der Merkmalsvektor der biometrischen Daten 33 zur Kategorie Ökonomische Daten umfasst beispielsweise die folgenden Merkmale:
a. Krankenstand
b. Arbeitsplatzrisiko

Die Kategorie Persönliche Daten umfasst Daten zum Alter und Familienstatus sowie zur Berufsgruppe und zum Bildungsstand. Der Merkmalsvektor der Kategorie Persönliche Daten wird insbesondere für eine individuelle Kalibrierung der aktuellen Belastungsniveaus 36, 36A, 36B, 36C, 36D herangezogen. Die Persönlichen Daten werden vom Benutzer beispielsweise über ein Formular innerhalb der weiteren Applikation 6 erfasst.

Der Merkmalsvektor der biometrischen Daten 33 zur Kategorie Persönliche Daten umfasst beispielsweise die folgenden Merkmale:
a. Berufsgruppe
b. Bildungsstand
c. Geoposition
d. Alter
e. Medikation
f. Vorerkrankungen
g. Erkrankungen in der Familie
h. Familienstatus

Die Fragebogen-Daten umfassen individuelle Selbsteinschätzungen des Benutzers zu belastungsrelevanten Fragestellungen. Die Fragebogen-Daten werden vom Benutzer beispielsweise über ein Formular innerhalb der weiteren Applikation 6 erfasst.

Die biometrischen Daten 33 zu den genannten weiteren Kategorien können zusätzlich zur Auswertung verwendet werden und insbesondere zur Ermittlung des konsolidierten aktuellen Belastungsniveaus 36 des Benutzers herangezogen werden.

Während in den beispielhaften Anwendungsfällen Schlaf und Motorik die Auswertung der biometrischen Daten 33 direkt durch die weitere Applikation 6 als Auswertungseinheit auf dem mobilen Endgerät 1 erfolgt, wurde für den beispielhaften Anwendungsfall Sprache eine andere Vorgehensweise gewählt. Um die Qualität des ermittelten dritten aktuellen Belastungsniveaus 36B sowie des konsolidierten aktuellen Belastungsniveau 36 zu erhöhen, erfolgt die Auswertung der biometrischen Daten 33 zur Kategorie Sprache in der Auswertungseinheit 13, die auf dem zentralen Server 10 angeordnet ist. In der Auswertungseinheit 13 ist ein Auswertungsverfahren vorgesehen, beispielsweise ein auf künstlichen neuronalen Netzen basierendes Verfahren, das auf biometrische Daten 33 anderer Benutzer und auf frühere biometrische Daten 33 des Benutzers zurückgreift.

In einer alternativen Ausführung der Erfindung werden auch die biometrischen Daten 33 zu anderen Kategorien in der auf dem zentralen Server 10 angeordneten Auswertungseinheit 13 ausgewertet, um die Qualität der Auswertung weiter zu erhöhen.

In einer wiederum alternativen Ausführung der Erfindung wird das auf dem zentralen Server 10 durch Training des künstlichen neuronalen Netz-Verfahrens gewonnene AuswertungsVerfahren in die weitere Applikation 6 implementiert, beispielsweise durch ein Update der weiteren Applikation 6. In diesem Fall ist die Auswertungseinheit für alle Kategorien durch die weitere Applikation 6 auf dem mobilen Endgerät 1 vorgesehen. Eine Auswertung der biometrischen Daten 33 zu allen Kategorien erfolgt auf dem mobilen Endgerät 1 und nicht auf dem zentralen Server 10.

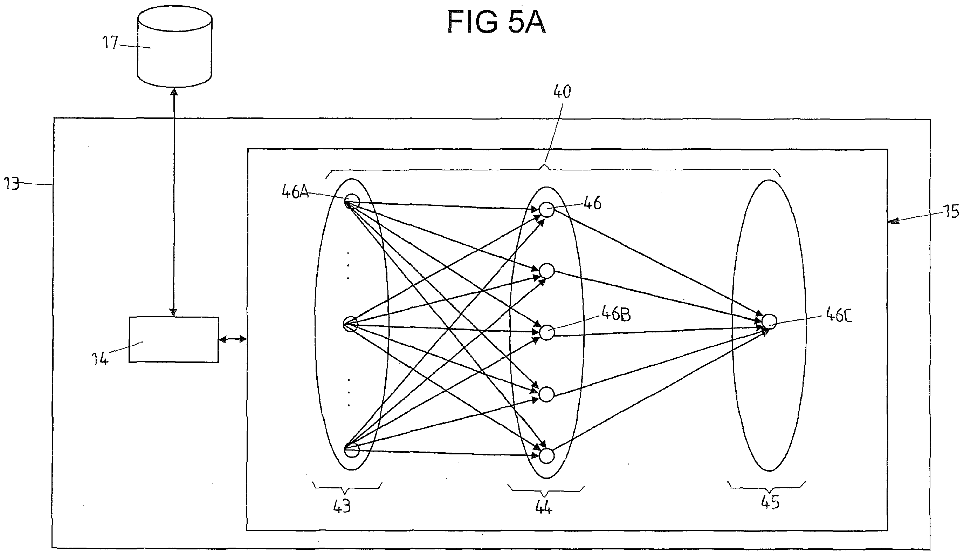

Figur 5A zeigt eine schematische Darstellung einer beispielhaften Ausführungsform der Auswertungseinheit 13 auf dem zentralen Server 10. In dieser Ausführungsform wird ein aktuelles Belastungsniveau 36, 36A, 36B, 36C, 36D zu einem Benutzer durch die Auswertungseinheit 13 auf dem zentralen Server 10 bestimmt.

In einer Variation dieser Ausführungsform kann aber auch ein Teil der biometrischen Daten 33 zum Benutzer direkt auf dem mobilen Endgerät 1 analysiert und ausgewertet werden und mindestens ein endgeräteseitig ermitteltes aktuelles Belastungsniveau 36A, 36B, 36C, 36D bestimmt werden. Ein zweiter Teil der biometrischen Daten 33 zum Benutzer wird auf dem zentralen Server 10 durch die Auswertungseinheit 13 analysiert und ausgewertet und mindestens ein serverseitiges aktuelles Belastungsniveau 36A, 36B, 36C, 36D bestimmt. Die serverseitig analysierten und ausgewerteten biometrischen Daten 33 können biometrische Daten 33 umfassen, die auch bei der Analyse und Auswertung auf dem mobilen Endgerät 1 berücksichtigt werden. Ein konsolidiertes Belastungsniveau 36, das sowohl das endgeräteseitig ermittelte mindestens eine aktuelle Belastungsniveau 36A, 36B, 36C, 36D als auch das serverseitig ermittelte mindestens eine aktuelle Belastungsniveau 36A, 36B, 36C, 36D berücksichtigt, wird durch den Datenmanager 61 der weiteren Applikation 6 bestimmt.

Die Auswertungseinheit 13 umfasst einen serverseitigen Datenmanager 14 und einen serverseitigen Datenanalysator 15. Der serverseitige Datenanalysator 15 ist als künstliches neuronales Netz 40 in Form eines Mehrschicht-Perceptron-Netzes ausgebildet. Das neuronale Netz besteht aus drei Schichten: der Eingabeschicht 43, der verborgenen Schicht 44 und der Ausgabeschicht 45. Jede Schicht ist aus Neuronen 46 aufgebaut. In der Eingabeschicht 43 ist eine Mehrzahl von Eingabe-Neuronen 46A angeordnet. In der verborgenen Schicht 44 ist eine Mehrzahl von verborgenen Neuronen 46B und in der Ausgabeschicht 45 ist genau ein Ausgabe-Neuron 46C angeordnet.

In einer möglichen Ausführungsform ist jedem Eingabe-Neuron 46A der Eingabeschicht 43 als Eingabewert der Wert eines Merkmals aus einem Merkmalsvektor einer Kategorie von biometrische Daten 33, die an den zentralen Server 10 übertragen wurden, beispielsweise der Kategorie Sprache, nach einer geeigneten Normalisierung, beispielsweise auf den Wertebereich [0,10], zugeordnet.

In einer alternativen Ausführungsform ist jedem Eingabe-Neuron 46A der Eingabeschicht 43 als Eingabewert das aktuelle Belastungsniveau zu einer Kategorie von biometrischen Daten 33 zugeordnet. Beispielsweise besteht die Eingabeschicht 43 aus sieben Eingabe-Neuronen 46A, wobei jedem Eingabe-Neuron 46A das aktuelle Belastungsniveau einer der Kategorien Schlaf, Sprache, Motorik, soziale Interaktion, ökonomische Daten, persönliche Daten und Fragebogen-Daten zugeordnet ist.

In einer weiteren alternativen Ausführungsform werden die Merkmale der kategoriespezifischen Merkmalsvektoren, der dem zentralen Server 10 vorliegenden biometrischen Daten 33, in einer anderen Weise verknüpft und ausgewertet, um die Eingabewerte der Eingabe-Neuronen 46A zu bestimmen.

Das Mehrschicht-Perceptron-Netz ist als Feedforward-Netz ausgebildet; d.h. die Verbindungen zwischen den Neuronen 46 weisen immer von einer Schicht, beispielsweise die Eingabeschicht 43, in die nächste Schicht, beispielsweise die Verborgene Schicht 44. Beim Durchlaufen des Neuronalen Netzes 40 kommt es zu keinen Rückkopplungen oder zyklischen Verbindungen, stattdessen werden Informationen nur in eine ausgezeichnete Richtung weitergegeben. Die Eingabe-Neuronen 46A der Eingabeschicht weisen Verbindungen zu den verborgenen Neuronen 46B der verborgenen Schicht auf. Beispielsweise kann jedes Eingabe-Neuron 46A der Eingabeschicht eine Verbindung zu jedem verborgenen Neuron 46B der verborgenen Schicht aufweisen. In einem Anfangszustand verfügt die verborgene Schicht 44 über eine höhere Anzahl von Neuronen 46 als die Eingabeschicht 43. Dagegen ist in der Ausgabeschicht 45 genau ein Neuron 46, das Ausgabe-Neuron 46C, angeordnet. Die Neuronen 46B der verborgenen Schicht 44 verfügen über Verbindungen zu dem Ausgabe-Neuron 46C der Ausgabeschicht 45. Beispielsweise ist jedes verborgene Neuron 46B der verborgenen Schicht 44 mit dem Ausgabe-Neuron 46C verbunden. Das Ausgabe-Neuron 46C repräsentiert ein aktuelles Belastungsniveau 36, 36A, 36B, 36C, 36D eines Benutzers.

Das künstliche neuronale Netz 40 berechnet aus den biometrischen Daten 33 eines Benutzers ein aktuelles Belastungsniveau 36, 36A, 36B, 36C, 36D des Benutzers. Dazu ruft der serverseitige Datenmanager 14 die biometrischen Daten 33 zu einem Benutzer in Form der Merkmalsvektoren zu den ermittelten und an den zentralen Server 10 übermittelten Kategorien von biometrischen Daten 33 aus der zentralen Speichereinheit 17 ab. Es finden die für die Berechnung des aktuellen Belastungsniveau 36, 36A, 36B, 36C, 36D geeigneten Merkmalsvektoren Berücksichtigung, beispielsweise die Merkmalsvektoren mit dem jüngsten Zeitstempel. Ein Merkmalsvektor einer Kategorie wird nur dann berücksichtigt, wenn der Zeitpunkt für den das aktuelle Belastungsniveau 36, 36A, 36B, 36C, 36D berechnet wird in dem durch den Zeitstempel definierten Gültigkeitsbereich liegt. Der serverseitige Datenmanager 14 stellt die biometrischen Daten 33 dem Datenanalysator 15 bereit, der als künstliches neuronales Netz 40 ausgebildet ist.

Die biometrischen Daten 33 werden, nach einer möglichen Aufbereitung, wie einer Normierung und/oder einer geeigneten Verknüpfung, in die Eingabeschicht 43 des neuronalen Netzes 40 eingelesen und über die Verbindungen in die nächsten Schichten des neuronalen Netzes 40 weitergegeben. Jede Verbindung weist ein Verbindungsgewicht auf, das entweder verstärkend oder hemmend wirkt. Jedes Neuron 46B der Verborgenen Schicht 44 verfügt über eine Aktivierungsfunktion, beispielsweise die Aktivierungsfunktion Tangens Hyperbolicus, welche einen beliebigen Eingangswert auf den Wertebereich [-1, 1] abbildet. Der Eingangswert eines Neurons 46B der Verborgenen Schicht 44 ergibt sich als Summe der über die gewichteten Verbindungen übertragenen Werte. Für jedes Neuron 46 ist ein neuronenspezifischer Schwellwert festgelegt. Überschreitet der Eingangswert, nach Anwendung der Aktivierungsfunktion, den Schwellwert des Neurons 46B, so wird dieser berechnete Wert vom Verborgenen Neuron 46B an seine ausgehenden Verbindungen und damit an das Ausgabe-Neuron 46C in der Ausgabeschicht 45 weitergegeben. Das Ausgabe-Neuron 46C bestimmt seinen Ausgabewert nach dem gleichen Verfahren, wie es für ein Verborgenes Neuron 46B der Verborgenen Schicht 44 beschrieben wurde. Für gegebene Verbindungsgewichte, Aktivierungsfunktionen und Schwellwerte bestimmt das künstliche neuronale Netz 40 aus den biometrischen Daten 33, die den Eingabe-Neuronen 46A zugeordnet werden, auf deterministische Weise den Wert des einen Ausgabe-Neurons 46C. Der Wert des Ausgabe-Neurons 46C liefert das aktuelle Belastungsniveau 36, 36A, 36B, 36C, 36D.

Der Wert des Ausgabe-Neurons 46C wird von dem als künstlichem neuronalen Netz 40 ausgebildeten serverseitigen Datenanalysator 15 an den serverseitigen Datenmanager 14 übergeben. Der serverseitige Datenmanager 14 speichert den Ausgabewert als aktuelles Belastungsniveau 36, 36A, 36B, 36C, 36D zu den für seine Bestimmung relevanten Kategorien mit einem Zeitstempel in der zentralen Speichereinheit 17.

In einer initialen Phase werden die Verbindungsgewichte jeder Verbindung und die Schwellwerte jedes Neurons 46 festgelegt. Beispielsweise werden die Verbindungsgewicht für eine Verbindung durch einen Zufallswert aus dem Intervall [-0.5 , 0,5] festgelegt, wobei der Wert 0 ausgespart wird. Der Schwellwert zu einem Neuron 46 wird beispielsweise durch einen Zufallswert aus dem Intervall [-0.5 , 0,5] festgelegt.

In einer Trainingsphase werden die Verbindungsgewichte jeder Verbindung des neuronalen Netzes 40 und die Schwellwerte zu jedem Neuron 46 angepasst. Zum Trainieren des neuronalen Netzes 40 wird ein überwachtes Lernverfahren, vorzugsweise ein Backpropagation-Verfahren, eingesetzt. Bei einem überwachten Lernverfahren liegt zu den Eingabewerten des Neuronalen Netzes 40 der gewünschte Ausgabewert des Ausgabe-Neurons 46C vor. Der gewünschte Ausgabewert des Ausgabe-Neurons 46C ergibt sich beispielsweise aus dem aktuellen Belastungsniveau zur Kategorie Fragebogen-Daten, welches ausschließlich aus den von dem Benutzer beantworteten Fragebogen-Daten ermittelt wurde. In einem iterativen Backpropagation-Verfahren werden die Verbindungsgewichte aller Verbindungen und die Schwellwerte aller Neuronen 46 solange trainiert bis der durch das neuronale Netz 40 gelieferte Ausgabewert des Ausgabe-Neurons 46C mit dem gewünschten Ausgabewert hinreichend genau übereinstimmt. Durch das wiederholte Training mit einer Vielzahl von biometrischen Daten 33 von einer Vielzahl von Benutzern kann das durch das künstliche neuronale Netz 40 gegebene Analyse- und Auswertungsverfahren zur Ermittlung des aktuellen Belastungsniveaus 36, 36A, 36B, 36C, 36D ständig weiter verbessert und angepasst werden.

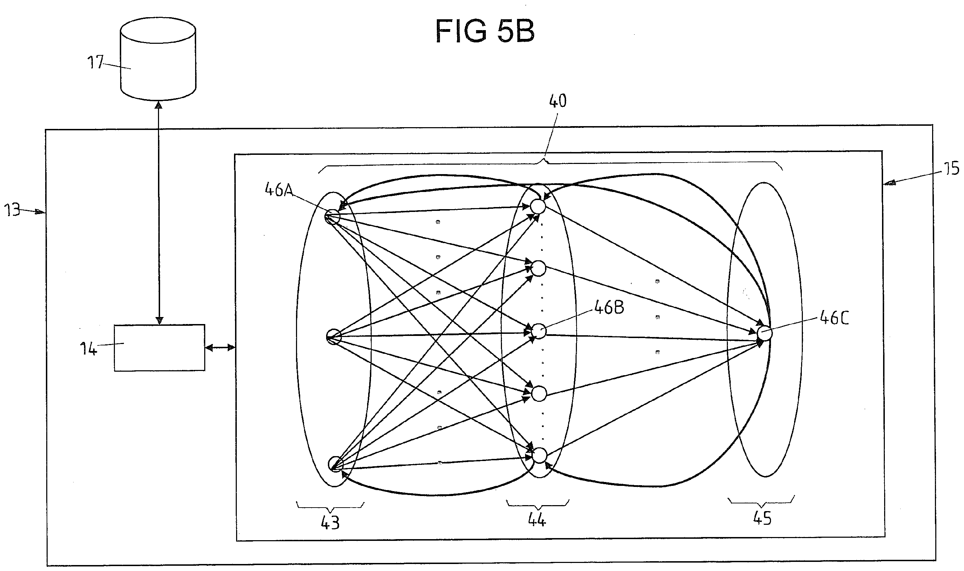

In Figur 5B findet sich eine schematische Darstellung einer alternativen beispielhaften Ausführungsform der Auswertungseinheit 13 auf dem zentralen Server 10. In dieser alternativen Ausführungsform ist das künstliche neuronale Netz 40 als rückgekoppeltes neuronales Netz ausgebildet. Neben den Verbindungen, die von einem Neuron 46 einer vorgelagerten Schicht zu einem Neuron 46 einer nachgelagerten Schicht weisen, beispielsweise von einem Eingabe-Neuron 46A der Eingabeschicht 43 zu einem Verborgenen Neuron 46B der verborgenen Schicht 44, weist diese Ausführungsform Verbindungen auf, die in umgekehrter Richtung verlaufen, beispielsweise von einem Verborgenen Neuron 46B der verborgenen Schicht 44 zu einem Eingabe-Neuron 46A der Eingabeschicht 43 oder von dem Ausgabe-Neuron 46C der Ausgabeschicht 45 zu einem verborgenen Neuron 46B der verborgenen Schicht 44. Ein derartiges künstliches neuronales Netz 40 weist eine höhere Komplexität auf, als das zuvor beschriebene Feedforward-Netz, welches Daten nur in einer ausgezeichneten Vorwärtsrichtung weitergibt.

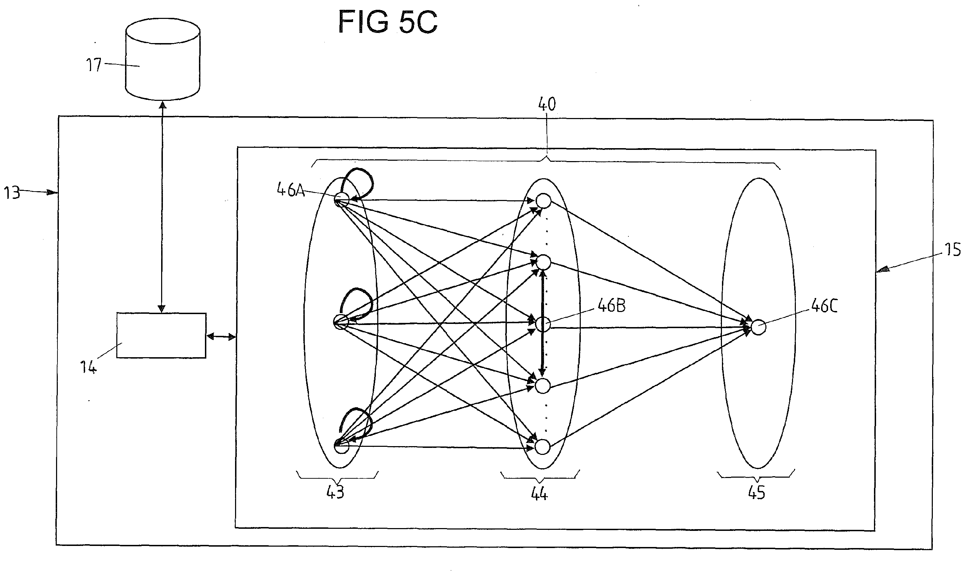

Eine Weiterbildung des rückgekoppelten künstlichen neuronalen Netzes zeigt Figur 5C. Danach sind auch laterale Rückkopplungen möglich, das heißt Verbindungen von Neuronen 46, die in derselben Schicht angeordnet sind. In einer weiteren Weiterbildung des rückgekoppelten künstlichen neuronalen Netzes ist auch eine direkte Rückkopplung vorgesehen. Bei einer direkten Rückkopplung handelt es sich um eine Verbindung von einem Neuron 46 zu sich selbst. Durch eine direkte Rückkopplung hemmen bzw. stärken Neuronen 46 sich selbst, um an Ihre Aktivierungsgrenzen zu gelangen.

Ein rückgekoppeltes künstliches neuronales Netz ist insbesondere vorgesehen, um das "Gedächtnis" von biometrischen Daten 33 zu einem Benutzer bei der Bestimmung des aktuellen Belastungsniveaus 36, 36A, 36B, 36C, 36D zu berücksichtigen. Bei dem Gedächtnis von biometrischen Daten 33 zu einer Kategorie zu einem Benutzer handelt es sich um die nach ihrem Zeitstempel angeordnete Folge von Merkmalsvektoren zu dieser Kategorie und diesem Benutzer, insbesondere umfasst die Folge ältere Merkmalsvektoren aus früheren Analysen. Es wird eine geeignete Teilfolge ausgewählt und das künstliche Neuronale Netz-Verfahren wird mit dem ersten Merkmalsvektor in dieser Teilfolge, also dem Merkmalsvektor mit dem ältesten Zeitstempel gestartet. In einem ersten Zeitschritt werden die Werte des ersten Merkmalsvektors als Eingabewerte an das künstliche Neuronale Netz angelegt und das Neuronale Netz wird einmal durchlaufen. Durch die eingebauten Rückkopplungen wirken die Werte des ersten Zeitschritts weiter auf den folgenden Zeitschritt. In dem folgenden Zeitschritt werden die Werte des zweiten Merkmalsvektors als Eingabewerte an das künstliche Neuronale Netz 40 angelegt. Beim erneuten Durchlaufen des künstlichen Neuronalen Netzes 40 werden zusätzlich zu den Eingabewerten des zweiten Merkmalsvektors die in rückgekoppelten Verbindungen generierten Werte aus dem vorherigen Zeitschritt als neue Eingabewerte berücksichtigt. Das derartig bestimmte Verfahren wird weiter fortgesetzt bis die komplette Teilfolge von Merkmalsvektoren durchlaufen ist. Der Wert des Ausgabe-Neurons 46C liefert das aktuelle Belastungsniveau 36, 36A, 36B, 36C, 36D des Benutzers.

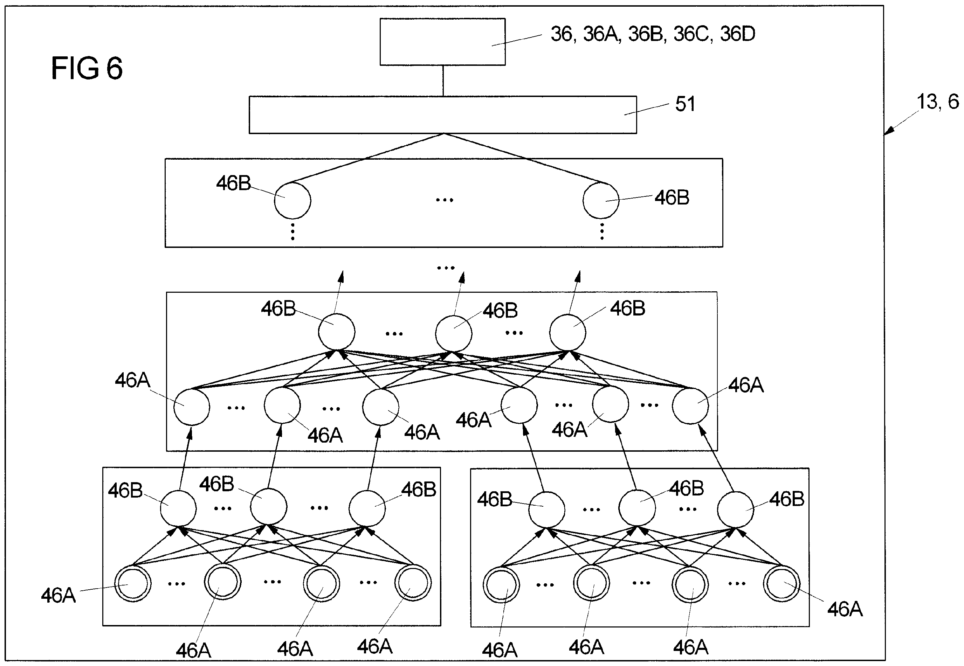

In Figur 6 findet sich eine schematische Darstellung der Ausführungsform der Erfindung. In dieser Ausführungsform ermittelt die Auswertungseinheit 6, 13 ein aktuelles Belastungsniveau 36, 36A, 36B, 36C, 36D eines Benutzers mit Hilfe eines Netzes von künstlichen neuronalen Netzen. Das Netz von neuronalen Netzen kann beispielsweise als ein Deep Belief Network oder ein Convolutional Deep Belief Network 50 ausgebildet sein. Ein einzelnes künstliches neuronales Netz 40, welches Teil des Netzes von künstlichen neuronalen Netzen ist, kann beispielsweise nach einer der für künstliche neuronale Netze 40 zuvor genannten Ausführungsformen ausgeführt sein.

Erfindungsgemäß, umfasst das Netz von künstlichen neuronalen Netzen eine Mehrzahl von künstlichen neuronalen Netzen 40, die miteinander interagieren. Beispielsweise kann die Mehrzahl von künstlichen neuronalen Netzen 40 als Restricted Boltzmann Machine oder als Convolutional Restricted Boltzmann Machine ausgeführt sein.

In dem Netz von künstlichen neuronalen Netzen 40 umfasst ein einzelnes neuronales Netz 40 eine Eingabeschicht 43 und eine verborgene Schicht 44. Die Eingabeschicht umfasst eine Mehrzahl von Eingabe-Neuronen 46A. Die verborgene Schicht umfasst eine Mehrzahl von verborgenen Neuronen 46B. Aus der Eingabeschicht eines künstlichen neuronalen Netzes ist die verborgene Schicht desselben neuronalen Netzes bestimmbar, so wie es beispielsweise in den vorhergehenden Ausführungsformen ausgeführt ist.

In dem Netz von künstlichen neuronalen Netzen ist eine erste Ebene von künstlichen neuronalen Netzen 40 vorgesehen, die als erste neuronale Netze bezeichnet seien. Die Eingabeschicht 43 der ersten neuronalen Netze wird durch die biometrischen Daten 33 des Benutzers festgelegt. Beispielsweise kann eine Komponente eines Merkmalsvektors einer Kategorie einem Eingabe-Neuron 46A zugeordnet werden. Darüber hinaus ist mindestens eine weitere Ebene von künstlichen neuronalen Netzen 40 vorgesehen, die als weitere neuronale Netze bezeichnet seien. Für ein weiteres neuronales Netz ist die Eingabeschicht 43 aus den verborgenen Schichten 44 einer Mehrzahl von künstlichen neuronalen Netzen 40 der vorausgehenden Ebene bestimmbar. Beispielsweise ist ein Eingabe-Neuron 46A der Eingabe schicht 43 durch genau ein verborgenes Neuron 46B eines künstlichen neuronalen Netzes 40 der vorhergehenden Schicht festgelegt. Oder ein Eingabe-Neuron 46A der Eingabe schicht 43 ist durch eine Mehrzahl von verborgenen Neuronen 46B eines oder einer Mehrzahl von künstlichen neuronalen Netzes 40 der vorhergehenden Schicht festgelegt.

In dem Netz von künstlichen neuronalen Netzen 40 ist eine oberste Ebene vorgesehen, die mindestens ein künstliches neuronales Netz 40 umfasst. Das mindestens eine künstliche neuronale Netz 40 der obersten Ebene sei als oberstes neuronales Netz bezeichnet.

Das mindestens eine oberste neuronale Netz verfügt über eine Ausgabeschicht 45. In einer ersten Ausführung wird die verborgene Schicht 44 eines obersten neuronalen Netzes mit der Ausgabeschicht 45 identifiziert. In einer zweiten Ausführung umfasst das mindestens eine künstliche neuronale Netz 40 der obersten Ebene drei Schichten, die Eingabeschicht, die verborgene Schicht und die Ausgabeschicht 45. In der zweiten Ausführung umfasst die Ausgabeschicht 45 genau ein Ausgabe-Neuron 46C.

Das aktuelle Belastungsniveau 36, 36A, 36B, 36C, 36D lässt sich aus der Ausgabeschicht 45 des mindestens einen obersten neuronalen Netzes bestimmen. In einer ersten Ausführung ist ein Klassifikator vorgesehen, der die Ausgabeschicht 45 klassifiziert und daraus das aktuelle Belastungsniveau 36, 36A, 36B, 36C, 36D bestimmt. Beispielsweise kann der Klassifikator als Support Vector Machine ausgelegt sein. In einer zweiten Ausführung ist das aktuelle Belastungsniveau 36, 36A, 36B, 36C, 36D durch das Ausgabe-Neuron 46C festgelegt.

Die Auswertungseinheit 6, 13, welche ein Netz von einer Mehrzahl von künstlichen neuronalen Netzen 40 umfasst, ist so ausgelegt, dass die Berechnung des Netzes parallelisiert ist.

Erfindungsgemäß, ist eine Mehrzahl von Prozessoren vorgesehen. Die Mehrzahl von Prozessoren kann auf dem mobilen Endgerät oder dem zentralen Server oder auf beiden vorgesehen sein. Die Auswertungseinheit 6, 13 ist ausgelegt und vorgesehen, die Mehrzahl von künstlichen neuronalen Netzen 40 durch die Mehrzahl von Prozessoren parallel berechnen zu lassen.

Durch die parallele Berechnung wird die Rechenzeit optimiert. Das auf einem Netz von künstlichen neuronalen Netzen basierende Verfahren zur Bestimmung eines aktuellen Belastungsniveaus lässt sich durch Parallelisierung der Berechnung von neuronalen Netzen schneller ausführen. Ebenso lässt sich durch die Parallelisierung Energie einsparen.

Erfindungsgemäß, wird zur Ausführung des Verfahrens mindestens eine Grafikkarte mit mindestens einem Grafikkartenprozessor eingebunden, wobei die mindestens eine Grafikkarte auf dem mobilen Endgerät oder auf dem zentralen Server angeordnet ist. Der Grafikkartenprozessor unterstützt die Berechnung der künstlichen neuronalen Netze. Durch dieses Vorgehen lässt sich die Rechenzeit noch weiter optimieren.

## Patentansprüche

1. Vorrichtung zur Ermittlung eines aktuellen Belastungsniveaus (36, 36A, 36B, 36C, 36D) eines Benutzers, umfassend
• ein mobiles Endgerät (1) mit
∘ mindestens einem Signaldaten (31) erzeugenden, in das mobile Endgerät (1) integrierten Sensor (2),
∘ einer Mehrzahl von verfügbaren Applikationen (5) zur Benutzung durch den Benutzer
und
• eine Auswertungseinheit (13, 6), die im mobilen Endgerät (1) oder in einem zentralen Server (10) vorgesehen ist,
wobei das mobile Endgerät (1) über eine weitere Applikation (6) verfügt, die ausgelegt ist, eine Mehrzahl biometrischer Daten (33) zum Benutzer wenigstens aus den Signaldaten (31) und aus vom Benutzer benutzten verfügbaren Applikationen (5) zu ermitteln und der Auswertungseinheit (13, 6) zur Verfügung zu stellen, und dass die Auswertungseinheit (13, 6) ausgelegt ist, das aktuelle Belastungsniveau (36, 36A, 36B, 36C, 36D) des Benutzers aus den biometrischen Daten (33) zu bestimmen,
**dadurch gekennzeichnet, dass**
die Auswertungseinheit (13, 6) ausgelegt ist, das aktuelle Belastungsniveau (36, 36A, 36B, 36C, 36D) des Benutzers aus den biometrischen Daten (33) durch Anwendung eines Verfahrens zu bestimmen, das auf einem Netz von künstlichen neuronalen Netzen (40) ausgeführt wird, welches eine Mehrzahl von künstlichen neuronalen Netzen (40) umfasst, die miteinander interagieren, und auf dem mobilen Endgerät (1) oder auf dem zentralen Server (10) eine Mehrzahl von Prozessoren angeordnet ist, die dazu ausgelegt sind die Mehrzahl von künstlichen neuronalen Netzen parallel zu berechnen und zusätzlich auf dem mobilen Endgerät (1) oder auf dem zentralen Server (10) mindestens eine Grafikkarte mit mindestens einem Grafikkartenprozessor angeordnet ist und der mindestens eine Grafikkartenprozessor die Berechnung der künstlichen neuronalen Netze (40) unterstützt.

2. Vorrichtung nach Anspruch 1, wobei die weitere Applikation (6) als SIM-Applikation im Speicherbereich auf einer SIM-Karte, die im mobilen Endgerät betrieben werden kann, speicherbar ist und von einer separaten auf der SIM-Karte integrierten Ausführungseinheit ausführbar ist.

3. Vorrichtung nach einem der Ansprüche 1 oder 2, wobei die Auswertungseinheit (13, 6) ausgelegt ist, das aktuelle Belastungsniveau (36, 36A, 36B, 36C, 36D) des Benutzers aus den biometrischen Daten (33) durch Anwendung eines Verfahrens zu bestimmen, das auf einer Mehrzahl von künstlichen neuronalen Netzen (40) ausgeführt wird, welche als ein Convolutional Deep Belief Network (50) ausgelegt sind.

4. Vorrichtung nach einem der vorherigen Ansprüche, wobei die Mehrzahl von biometrischen Daten (33) in eine Mehrzahl von Kategorien unterteilbar ist, wobei die Kategorien beispielsweise mehrere der folgenden Kategorien umfassen
a. Schlaf,
b. Sprache,
c. Motorik,
d. Soziale Interaktion,
e. Ökonomische Daten,
f. Persönliche Daten,
g. Fragebogen-Daten.

5. Vorrichtung nach einem der vorherigen Ansprüche, wobei jedes künstliche neuronale Netz (40) zwei Neuronen-Schichten, eine Eingabeschicht (43) und eine verborgene Schicht (44) umfasst, wobei die Eingabeschicht (43) eine Mehrzahl von Eingabe-Neuronen (46A) und die verborgene Schicht (44) eine Mehrzahl von verborgenen Neuronen (46B) umfasst, und wobei die Eingabe-Neuronen durch die Mehrzahl von biometrischen Daten festlegbar sind.

6. Vorrichtung nach Anspruch 5, wobei die Auswertungseinheit (13, 6) ausgelegt ist, die Eingabeschicht (43) von mindestens einem der künstlichen neuronalen Netze (40) durch die verborgenen Schichten (44) von einer Mehrzahl von anderen künstlichen neuronalen Netzen festzulegen.

7. Vorrichtung nach einem der Ansprüche 5 oder 6, wobei die Auswertungseinheit (13, 6) ausgelegt ist, das aktuelle Belastungsniveau (36, 36A, 36B, 36C, 36D) aus mindestens einem Ausgabe-Neuron (46C) festzulegen, das mit mindestens einem verborgenen Neuron mindestens eines künstlichen neuronalen Netzes (40) identifizierbar ist.

8. Vorrichtung nach einem der Ansprüche 5 bis 7, wobei die Auswertungseinheit (13, 6) mit einer Mehrzahl von Prozessoren zusammenwirkt und der jeweilige Prozessor ausgelegt ist, Neuronen (46, 46B, 46C) zu mindestens einem der Mehrzahl von künstlichen neuronalen Netzen (40) zu berechnen.

9. Vorrichtung nach einem der vorherigen Ansprüche, wobei der mindestens eine im mobilen Endgerät (1) integrierte Sensor (2) ein Gyroskop (21), einen Beschleunigungssensor (22), einen Lichtsensor (23) oder eine Kombination dieser Sensoren umfasst.

10. Verfahren zur Ermittlung eines aktuellen Belastungsniveaus (36, 36A, 36B, 36C, 36D) eines Benutzers eines mobilen Endgerätes (1), umfassend die Schritte
• Starten einer auf einem mobilen Endgerät (1) installierten weiteren Applikation (6), so dass diese auf dem mobilen Endgerät (1) ausgeführt wird,
• Ermitteln einer Mehrzahl biometrischer Daten (33) zum Benutzer durch die weitere Applikation (6), wobei die biometrischen Daten (33) wenigstens aus Benutzungsdaten, die aus der Benutzung von mindestens einer auf dem mobilen Endgerät (1) verfügbaren vorhandenen Applikation (5) durch den Benutzer erfasst werden, und aus Signaldaten (31) von mindestens einem in das mobile Endgerät (1) integrierten Sensor (2) ermittelt werden,
• Auswerten der biometrischen Daten (33) durch eine im mobilen Endgerät (1) oder in einem zentralen Server (10) vorgesehene Auswertungseinheit (13, 6) zur Bestimmung des aktuellen Belastungsniveaus (36, 36A, 36B, 36C, 36D),
**dadurch gekennzeichnet, dass**
• die Auswertungseinheit (13, 6) das aktuelle Belastungsniveau (36, 36A, 36B, 36C, 36D) mit Hilfe von einem Netz von künstlichen neuronalen Netzen (40) bestimmt, wobei das Netz von künstlichen neuronalen Netzen (40) eine Mehrzahl von künstlichen neuronalen Netzen (40) umfasst, die miteinander interagieren, dabei wird
- die Mehrzahl von künstlichen neuronalen Netzen durch eine Mehrzahl von Prozessoren parallel berechnet, wobei die Mehrzahl von Prozessoren auf dem mobilen Endgerät (1) oder auf dem zentralen Server (10) angeordnet ist, und zusätzlich
- unterstützt mindestens ein Grafikkartenprozessor von mindestens einer Grafikkarte die Berechnung der künstlichen neuronalen Netze (40), wobei die mindestens eine Grafikkarte mit dem mindestens einen Grafikkartenprozessor auf dem mobilen Endgerät (1) oder auf dem zentralen Server (10) angeordnet ist.

11. Verfahren nach Anspruch 10, welches auf einer Vorrichtung nach einem der Ansprüche 1 bis 9 durchführbar ist.

12. Verfahren nach einem der Ansprüche 10 oder 11, wobei die weitere Applikation (6), solange sie auf dem mobilen Endgerät (1) ausgeführt wird, zum Ermitteln der biometrischen Daten (33) verifiziert, dass wenigstens von dem mindestens einen in das mobile Endgerät (1) integrierten Sensor (2) Signaldaten (31) bereitgestellt werden oder von der mindestens einen auf dem mobile Endgerät (1) verfügbaren Applikation (5) Benutzungsdaten bereitstellt werden, und die weitere Applikation (6) bei erfolgreicher Verifikation die biometrischen Daten (33) wenigstens aus den bereitgestellten Signaldaten (31) oder den bereitgestellten Benutzungsdaten ermittelt und mit einem Zeitstempel versieht und wobei die Auswertungseinheit (13, 6) das aktuelle Belastungsniveaus (36, 36A, 36B, 36C, 36D) aus den biometrische Daten (33) bestimmt, deren Zeitstempel aktuell gültig ist.

13. Verfahren nach einem der Ansprüche 10 bis 12, wobei die Auswertungseinheit (13, 6) jedes künstliche neuronale Netz (40) auf Basis von biometrischen Daten (33) und von zu den biometrischen Daten (33) bestimmten evaluierten Belastungsniveaus (36E) trainiert, wodurch die Qualität des künstlichen neuronalen Netzes (40) verbessert wird.

14. Applikation für ein mobiles Endgerät (1), die auf einem computerverwendbaren Medium speicherbar ist, umfassend computerlesbare Programmmittel, **dadurch gekennzeichnet, dass** sie mindestens einen Prozessor des Endgerätes (1) veranlasst, ein Verfahren nach einem der Ansprüche 10 bis 13 auszuführen.

## Claims

1. Device for calculating a current load level (36, 36A, 36B, 36C, 36D) of a user, comprising
• a mobile end unit (1) with
∘ at least one signal data (31) generating sensor (2) integrated into the mobile end unit (1),
∘ a multitude of available applications (5) for use by the user
and
• an evaluation unit (13, 6) provided in the mobile end unit (1) or in a central server (10),
wherein the mobile end unit (1) has a further application (6), designed for calculating a multitude of biometric data (33) about the user, at least from the signal data (31), and
from available applications (5) used by the user, and making these available to the evaluation unit (13, 6), and in that the evaluation unit (13, 6) is designed for determining the current load level (36, 36A, 36B, 36C, 36D) of the user from the biometric data (33),
**characterised in that**
the evaluation unit (13, 6) is designed for determining the current load level (36, 36A, 36B, 36C, 36D) of the user from the biometric data (33) by applying a method carried out in a network of artificial neural networks (40), which comprises a multitude of artificial neural networks (40) that interact with each other, and that a multitude of processors are arranged on the mobile end unit (1) or on the central server (10), which are designed for calculating the multitude of artificial neural networks in parallel, and that at least one graphics card with at least one graphics card processor is arranged on the mobile end unit (1) or on the central server (10) and the at least one graphics card processor supports the calculation of the artificial neural networks (40).

2. Device according to claim 1, wherein the further application (6) can be stored as a SIM application in the memory area on a SIM card that can be operated in the mobile end unit and can be executed by a separate execution unit integrated on the SIM card.

3. Device according to any of the claims 1 or 2, wherein the evaluation unit (13, 6) is designed for determining the current load level (36, 36A, 36B, 36C, 36D) of the user from the biometric data (33) by using a method that is carried out on a multitude of artificial neural networks (40), which are designed as a Convolutional Deep Belief Network (50).

4. Device according to any of the preceding claims, wherein the multitude of biometric data (33) can be divided into a multitude of categories, wherein the categories for example comprise several of the following categories:
a. sleep,
b. speech,
c. motor skills,
d. social interaction,
e. economic data,
f. personal information,
g. questionnaire data.

5. Device according to any of the preceding claims, wherein each artificial neural network (40) comprises two neuron layers, one input layer (43) and one hidden layer (44), wherein the input layer (43) comprises a multitude of input neurons (46A) and the hidden layer (44) a multitude of hidden neurons (46B), and wherein the input neurons can be determined by the multitude of biometric data.

6. Device according to claim 5, wherein
the evaluation unit (13, 6) is designed for determining the input layer (43) of at least one of the artificial neural networks (40) through the hidden layers (44) of a multitude of other artificial neural networks.

7. Device according to any of the claims 5 or 6, wherein the evaluation unit (13, 6) is designed for determining the current load level (36, 36A, 36B, 36C, 36D) from at least one output neuron (46C), which is identifiable with at least one hidden neuron of at least one artificial neural network (40).

8. Device according to any of the claims 5 to 7, wherein the evaluation unit (13, 6) cooperates with a multitude of processors and the respective processor is designed for calculating neurons (46, 46B, 46C) for at least one of the multitudes of artificial neural networks (40).

9. Device according to any of the preceding claims, wherein the at least one sensor (2) integrated into the mobile end unit (1) comprises a gyroscope (21), an acceleration sensor (22), a light sensor (23) or a combination of these sensors.

10. Method for calculating a current load level (36, 36A, 36B, 36C, 36D) of a user of a mobile end unit (1), comprising the steps:
• starting a further application (6) installed on a mobile end unit (1) so that this is carried out on the mobile end unit (1),
• calculating a multitude of biometric data (33) of the user by means of the further application (6), wherein the biometric data (33) is recorded at least from user data that is recorded from using at least one application (5) present and available on the mobile end unit (1) by the user, and calculated from signal data (31) of at least one sensor (2) integrated into the mobile end unit (1),
• evaluating the biometric data (33) with an evaluation unit (13, 6) provided in the mobile end unit (1) or in a central server (10) for determining the current load level (36, 36A, 36B, 36C, 36D),
**characterised in that**
• the evaluation unit (13, 6) determines the current load level (36, 36A, 36B, 36C, 36D) with the aid of a network of artificial neural networks (40), wherein the network of artificial neural networks (40) comprises a multitude of artificial neural networks (40) that interact with each other, where
- the multitude of artificial neural networks calculates in parallel with a multitude of processors, wherein the multitude of processors is arranged on the mobile end unit (1) or on the central server (10), and additionally
- at least one graphics card processor of at least one graphics card supports the calculation of the artificial neural networks (40), wherein the at least one graphics card with the at least one graphics card processor is arranged on the mobile end unit (1) or on the central server (10).

11. Method according to claim 10, which can be carried out on a device according to any of the claims 1 to 9.

12. Method according to any of the claims 10 or 11, wherein the further application (6), as long as it is carried out on the mobile end unit (1), verifies that signal data (31) is provided at least by the at least one sensor (2) integrated into the mobile end unit (1), or that usage data is provided by the at least one application (5) available on the mobile end unit (1) for calculating the biometric data (33), and the further application (6) calculates the biometric data (33) at least from the provided signal data (31) or the provided usage data during a successful verification and equips this with a time stamp, and wherein the evaluation unit (13, 6) determines the current load level (36, 36A, 36B, 36C, 36D) from the biometric data (33), the time stamp of which is currently valid.

13. Method according to any of the claims 10 to 12, wherein the evaluation unit (13, 6) trains each artificial neural network (40) on the basis of biometric data (33) and from load levels (36E) evaluated for the biometric data (33) determined, which improves the quality of the artificial neural network (40).

14. Application for a mobile end unit (1), which can be stored on a computer operatable medium, comprising computer readable program resources, **characterised in that** it initiates at least one processor of the end unit (1) to carry out a method according to any of the claims 10 to 13.

## Revendications

1. Dispositif pour déterminer un niveau de contrainte actuel (36, 36A, 36B, 36C, 36D) d'un utilisateur, comprenant
• un terminal mobile (1) avec
∘ au moins un capteur (2) intégré dans le terminal mobile (1) générant des données de signal (31),
∘ une multitude d'applications (5) disponibles pour être utilisées par l'utilisateur, et
• une unité d'évaluation (13, 6), qui est prévue dans le terminal mobile (1) ou dans un serveur central (10), le terminal mobile (1) disposant d'une autre application (6) qui est conçue afin de déterminer une multitude de données biométriques (33) pour l'utilisateur au moins à partir des données de signal (31) et des applications (5) disponibles utilisées par l'utilisateur et de les mettre à la disposition de l'unité d'évaluation (13, 6), et l'unité d'évaluation (13, 6) est conçue afin de déterminer le niveau de contrainte actuel (36, 36A, 36B, 36C, 36D) de l'utilisateur à partir des données biométriques (33), **caractérisé en ce que** l'unité d'évaluation (13, 6) est conçue afin de déterminer le niveau de contrainte actuel (36, 36A, 36B, 36C, 36D) de l'utilisateur à partir des données biométriques (33) en utilisant un procédé qui est réalisé sur un réseau de réseaux neuronaux artificiels (40) qui comprend une multitude de réseaux neuronaux artificiels (40) qui interagissent, et une multitude de processeurs est disposée sur le terminal mobile (1) ou sur le serveur central (10), processeurs qui sont conçus afin de calculer en parallèle la multitude de réseaux neuronaux artificiels, et au moins une carte graphique est également disposée avec au moins un processeur à carte graphique sur le terminal mobile (1) ou sur le serveur central (10) et l'au moins un processeur à carte graphique prend en charge le calcul des réseaux neuronaux artificiels (40).

2. Dispositif selon la revendication 1, l'autre application (6) pouvant être enregistrée en tant qu'application SIM dans la zone de mémoire sur une carte SIM qui peut être exploitée dans le terminal mobile et étant exécutable par une unité d'exécution séparée intégrée sur la carte SIM.

3. Dispositif selon l'une quelconque des revendications 1 ou 2, l'unité d'évaluation (13, 6) étant conçue afin de déterminer le niveau de contrainte actuel (36, 36A, 36B, 36C, 36D) de l'utilisateur à partir des données biométriques (33) en utilisant un procédé qui est réalisé sur une multitude de réseaux neuronaux artificiels (40) qui sont conçus comme un Convolutional Deep Belief Network (50).

4. Dispositif selon l'une quelconque des revendications précédentes, la multitude de données biométriques (33) pouvant être sous-divisée en une multitude de catégories, les catégories comprenant, par exemple, plusieurs des catégories suivantes
a. sommeil,
b. langue,
c. motricité,
d. interaction sociale,
e. données économiques,
f. données personnelles,
g. données collectées dans un questionnaire.

5. Dispositif selon l'une quelconque des revendications précédentes, chaque réseau neuronal artificiel (40) comprenant deux couches de neurones, une couche d'entrée (43) et une couche cachée (44), la couche d'entrée (43) comprenant une multitude de neurones d'entrées (46A) et la couche cachée (44) une multitude de neurones cachés (46B), et les neurones d'entrées pouvant être définis par la multitude de données biométriques.

6. Dispositif selon la revendication 5, l'unité d'évaluation (13, 6) étant conçue afin de déterminer la couche d'entrée (43) d'au moins un des réseaux neuronaux artificiels (40) par les couches cachées (44) d'une multitude d'autres réseaux neuronaux artificiels.

7. Dispositif selon l'une quelconque des revendications 5 ou 6, l'unité d'évaluation (13, 6) étant conçue afin de déterminer le niveau de contrainte actuel (36, 36A, 36B, 36C, 36D) à partir d'au moins un neurone de sortie (46C) qui soit identifiable avec au moins un neurone caché d'au moins un réseau neuronal artificiel (40).

8. Dispositif selon l'une quelconque des revendications 5 à 7, l'unité d'évaluation (13, 6) interagissant avec une multitude de processeurs et le processeur respectif étant conçu afin de calculer des neurones (46, 46B, 46C) parmi au moins un de la multitude de réseaux neuronaux artificiels (40).

9. Dispositif selon l'une quelconque des revendications précédentes, l'au moins un capteur (2) intégré dans le terminal mobile (1) comprenant un gyroscope (21), un capteur d'accélération (22), un capteur de lumière (23) ou une combinaison de ces capteurs.

10. Procédé pour déterminer un niveau de contrainte actuel (36, 36A, 36B, 36C, 36D) d'un utilisateur d'un terminal mobile (1), comprenant les étapes suivantes
• démarrage d'une autre application (6) installée sur un terminal mobile (1) de manière à ce que celle-ci soit réalisée sur le terminal mobile (1),
• détermination d'une multitude de données biométriques (33) sur l'utilisateur par l'autre application (6), les données biométriques (33) étant déterminées au moins à partir de données d'utilisation étant saisies par l'utilisateur selon l'utilisation d'au moins une application (5) présente et mise à disposition sur le terminal mobile (1), et à partir de données de signal (31) d'au moins un capteur (2) intégré dans le terminal mobile (1),
• évaluation des données biométriques (33) par une unité d'évaluation (13, 6) prévue dans le terminal mobile (1) ou dans un serveur central (10) afin de déterminer le niveau de contrainte actuel (36, 36A, 36B, 36C, 36D), **caractérisé en ce que**
• l'unité d'évaluation (13, 6) détermine le niveau de contrainte actuel (36, 36A, 36B, 36C, 36D) à l'aide d'un réseau de réseaux neuronaux artificiels (40), le réseau de réseaux neuronaux artificiels (40) comprenant une multitude de réseaux neuronaux artificiels (40) qui interagissent entre eux,
- la multitude de réseaux neuronaux artificiels étant calculée en parallèle par une multitude de processeurs, la multitude de processeurs étant disposée sur le terminal mobile (1) ou sur le serveur central (10), et **en ce**
- **qu'**au moins un processeur à carte graphique d'au moins une carte graphique assiste également le calcul des réseaux neuronaux artificiels (40), l'au moins une carte graphique étant disposée avec l'au moins un processeur à carte graphique sur le terminal mobile (1) ou sur le serveur central (10).

11. Procédé selon la revendication 10, qui est réalisable sur un dispositif selon l'une quelconque des revendications 1 à 9.

12. Procédé selon l'une quelconque des revendications 10 ou 11, l'autre application (6) vérifiant, tant qu'elle est exécutée sur le terminal mobile (1) afin de déterminer les données biométriques (33), qu'au moins des données de signal (31) sont fournies par l'au moins un capteur (2) intégré dans le terminal mobile (1) ou que des données d'utilisation sont fournies par l'au moins une application (5) disponible sur le terminal mobile (1), et que l'autre application (6) détermine, une fois la vérification terminée, les données biométriques (33) au moins à partir des données de signal (31) fournies ou des données d'utilisation fournies et appose un cachet de datation, et l'unité d'évaluation (13, 6) déterminant le niveau de contrainte actuel (36, 36A, 36B, 36C, 36D) provenant des données biométriques (33) dont le cachet de datation est actuellement valable.

13. Procédé selon l'une quelconque des revendications 10 à 12, l'unité d'évaluation (13, 6) formant chaque réseau neuronal artificiel (40) sur la base de données biométriques (33) et de niveaux de contrainte (36E) évalués et déterminés avec les données biométriques (33) améliorant ainsi la qualité du réseau neuronal artificiel (40).

14. Application destinée à un terminal mobile (1) qui peut être enregistrée sur un support utilisable sur un ordinateur, comprenant des moyens de programme lisibles par ordinateur, **caractérisée en ce qu'**elle amène au moins un processeur du terminal (1) à réaliser un procédé selon l'une quelconque des revendications 10 à 13.
